# EUROPEAN PATENT APPLICATION

(11) **EP 4 616 943 A1**
(43) Date of publication of application: **17.09.2025**
(21) Application number: 23888731.9
(22) Date of filing: 08.11.2023
(51) Int. Cl.: B01J 29/40, B01J 29/87, B01J 29/88, C07B 61/00, C07C 5/02, C07C 9/14

(54) **ZEOLITE COMPOSITE CATALYST, AND METHOD FOR PERFORMING HYDROGENOLYSIS OF CYCLIC COMPOUND HAVING FIVE-MEMBER RING STRUCTURE USING SAME**

(30) Priority: 09.11.2022 JP 2022179575
(71) Applicant: Chiyoda Corporation, Kanagawa 220-8765 (JP)
(72) Inventor: HODOSHIMA Shinya, Yokohama-shi, Kanagawa 220-8765 (JP); IMAGAWA Kenichi, Yokohama-shi, Kanagawa 220-8765 (JP); MOTOMIYA Azusa, Yokohama-shi, Kanagawa 220-8765 (JP)
(74) Representative: Ipsilon Benelux
(86) International application number: PCT/JP2023/040180
(87) International publication number: WO 2024/101384

(57) **Abstract**

[Problem] To selectively hydrocrack a cyclic compound having a five-membered ring structure included in an aromatic compound used as a hydrogen carrier.

[Solution] A zeolite composite catalyst used for hydrocracking a cyclic compound having a five-membered ring structure, the five-membered ring structure being included in an aromatic compound used as a hydrogen carrier, wherein the zeolite composite catalyst comprises aluminum or gallium, and wherein the zeolite composite catalyst comprises a zeolite with an MFI structure, and silica as a binder.

## Description

### TECHNICAL FIELD

The present invention relates to a zeolite composite catalyst used for hydrocracking a cyclic compound having a five-membered ring structure and a method for hydrocracking a cyclic compound having a five-membered ring structure using the same.

### BACKGROUND ART

Known methods for storing and transporting hydrogen include the organic chemical hydride method (OCH Method; Non-Patent Documents 1-3). The organic chemical hydride method involves a hydrogenation reaction (hydrogen storage reaction) in which an aromatic compound(s) such as toluene react with hydrogen, and a dehydrogenation reaction (hydrogen generation reaction) in which hydrogen is generated from a saturated cyclic compound(s) (hydrogenated aromatic compound) such as methylcyclohexane to recover the aromatic compound(s) such as toluene. In this method, a saturated cyclic compound such as methylcyclohexane and an aromatic compound such as toluene, which are produced after hydrogen is extracted from the saturated cyclic compound, are repeatedly used as containers for hydrogen (hydrogen carriers).

It has been well known that hydrogenation and dehydrogenation reactions in the organic chemical hydride method produce various by-products (impurities) other than target products (Patent Document 1).

### PRIOR ART DOCUMENT(S)

### PATENT DOCUMENT(S)

[Non-Patent Document 1]
   Yoshimi Okada, Energy and Resources, Vol. 33, No. 3, 168 (2018)
[Non-Patent Document 2]
   Yoshimi Okada, Tokyo High Pressure Gas Association Bulletin, August and September 2019
[Non-Patent Document 3]
   Xieli Sai, Mika Ishii, Tetsuya Namba, Taku Tsujimura, Takaaki Taniguchi, 46th Petroleum and Petrochemical Symposium, Lecture Abstract, 2A03 (2016)
[Patent Document 1]
   JP2007-269522A

### SUMMARY OF THE INVENTION

### TASK TO BE ACCOMPLISHED BY THE INVENTION

Thus, in the organic chemical hydride method, repeated hydrogenation and dehydrogenation reactions gradually increase the rate of impurities in the hydrogen carrier. One possible approach to addressing this problem is to distill the hydrogen carrier containing impurities to thereby remove (separate) such impurities from the hydrogen carrier.

Impurities that can be mixed into the hydrogen carrier include cyclic compounds with a five-membered ring structure (herein also referred to as "five-membered ring compounds") such as cyclopentanes, which are produced in the dehydrogenation reaction. Some of five-membered ring compounds, in particular, those with seven carbon atoms (such as dimethylcyclopentanes and ethylcyclopentanes), generally have their boiling points that are relatively close to the boiling point of the hydrogen carrier, which makes it difficult to separate these five-membered ring compounds from the hydrogen carrier by distillation.

As a result of efforts to address this problem, the inventors have developed a zeolite composite catalyst used for selectively hydrocracking five-membered ring compounds mixed in the hydrogen carrier, and found that use of the developed zeolite composite catalyst enables easy separation of the five-membered ring compounds (hydrocracked light hydrocarbons) from the hydrogen carrier.

The present invention has been made based on the above-described problem of the prior art, and a primary object of the present invention is to provide a zeolite composite catalyst used for hydrocracking a cyclic compound having a five-membered ring structure and a method for hydrocracking a cyclic compound having a five-membered ring structure using the same.

### MEANS TO ACCOMPLISH THE TASK

As a solution to the above-described tasks to be accomplished, an aspect of the present invention provides a zeolite composite catalyst used for hydrocracking a cyclic compound having a five-membered ring structure, the five-membered ring structure being included in an aromatic compound used as a hydrogen carrier, wherein the zeolite composite catalyst comprises at least one of aluminum, gallium, and iron, and wherein the zeolite composite catalyst comprises a zeolite with an MFI structure, and silica.

This configuration enables selective hydrocracking of a cyclic compound having a five-membered ring structure included in an aromatic compound used as a hydrogen carrier. In other words, in a process of hydrocracking a cyclic compound having a five-membered ring structure mixed in a hydrogen carrier (in particular, an aromatic compound such as toluene), this configuration enables the selective reaction while minimizing the loss of the hydrogen carrier (unintended chemical reaction).

The above zeolite composite catalyst may be further configured such that the zeolite with the MFI structure comprises gallium, and has an acid density of 5 to 200 where the acid density is determined as a ratio of an amount of silicon to an amount of gallium, the amounts being expressed in moles.

In this configuration, the acid density, which is a ratio of an amount of silicon to an amount of gallium, the amounts being expressed in moles, is set in a proper range, which enables effective hydrocracking of a cyclic compound having a five-membered ring structure while minimizing the loss of the hydrogen carrier.

The above zeolite composite catalyst may be further configured such that the zeolite with the MFI structure comprises iron and aluminum.

In this configuration, the zeolite with the MFI structure comprises proper metals, which enables stable hydrocracking of a cyclic compound having a five-membered ring structure.

The above zeolite composite catalyst may be further configured such that the zeolite has an acid density of 5 to 200 where the acid density is determined as a ratio of an amount of silicon to a sum of amounts of iron and aluminum, and also has an iron ratio of 0.1 to 0.9 where the iron ratio is a ratio of an amount of iron to a sum of amounts of iron and aluminum, the amounts being expressed in moles.

In this configuration, the acid density, which is a ratio of an amount of silicon to a sum of amounts of iron and aluminum, and the iron ratio, which is a ratio of an amount of iron to a sum of amounts of iron and aluminum, the amounts being expressed in moles, are set in their proper ranges, which enables effective hydrocracking of a cyclic compound having a five-membered ring structure while minimizing the loss of the hydrogen carrier.

The above zeolite composite catalyst may be further configured such that the zeolite with the MFI structure comprises aluminum, and has an acid density of 5 to 200 where the acid density is determined as a ratio of an amount of silicon to an amount of aluminum, the amounts being expressed in moles.

In this configuration, the acid density, which is a ratio of an amount of silicon to an amount of aluminum, the amounts being expressed in moles, is set in a proper range, which enables effective hydrocracking of a cyclic compound having a five-membered ring structure while minimizing the loss of the hydrogen carrier.

As a solution to the above-described tasks to be accomplished, another aspect of the present invention provides a method for hydrocracking a cyclic compound having a five-membered ring structure, the five-membered ring structure being included in an aromatic compound used as a hydrogen carrier, using the above zeolite composite catalyst, the method comprising: causing dehydrogenation of a saturated cyclic compound to produce, as reaction products, the aromatic compound and a cyclic compound having a five-membered ring structure as an impurity of the aromatic compound; and hydrocracking at least part of the reaction products, using the zeolite composite catalyst.

In a system in which the organic chemical hydride method is carried out, this configuration enables selective hydrocracking of a cyclic compound having a five-membered ring structure included in an aromatic compound used as a hydrogen carrier.

The above method may be further configured such that, during a reaction of the hydrocracking, a temperature of a catalyst bed is adjusted to 200°C to 450°C.

This configuration, in which a temperature of a catalyst bed is properly adjusted, can cause stable hydrocracking reactions.

The above method may be further configured such that the method further comprises separating gas and liquid phases of the reaction products to produce a liquid phase product, and wherein the step of hydrocracking using the zeolite composite catalyst comprises hydrocracking a mixture of the liquid phase product and hydrogen.

This configuration involves separating gas and liquid phases of the reaction products to produce a liquid phase product, and hydrocracking the liquid phase product, which enables stable hydrocracking reactions.

The above method may be further configured such that the mixture contains hydrogen in an amount ranging from 5 mol% to 90 mol%.

This configuration can cause hydrocracking reactions in the mixture in a more stable manner.

### EFFECT OF THE INVENTION

The present invention embodied as described above, enables selective hydrocracking of a cyclic compound having a five-membered ring structure included in an aromatic compound used as a hydrogen carrier.

### BRIEF DESCRIPTION OF THE DRAWINGS

[Figure 1] Figure 1 is a block diagram showing a schematic configuration of a hydrogen storage and transportation system according to a first embodiment of the present invention;
[Figure 2] Figure 2 is a diagram showing by-products in the dehydrogenation reaction of methylcyclohexane and their boiling points;
[Figure 3] Figure 3 is a diagram showing by-products in the dehydrogenation reaction of cyclohexane and their boiling points;
[Figure 4] Figure 4 is an explanatory diagram showing hydrocracking of methylcyclopentane;
[Figure 5] Figure 5 includes charts showing effect test results (i.e., results of tests for the effects) of zeolite composite catalysts according to the first embodiments (the charts for catalyst layer temperatures at (A) 350°C, (B) 375°C, and (C) 400°C);
[Figure 6] Figure 6 includes a chart showing the effect of hydrogen concentration in a raw material on hydrocracking with the use of Fe-Al-MFI/SiO2 catalyst according to the first embodiment (the chart for a catalyst layer temperature at 350°C);
[Figure 7] Figure 7 is a block diagram showing a schematic configuration of a hydrogen storage and transportation system according to a second embodiment of the present invention;
[Figure 8] Figure 8 includes charts showing the effect of LHSV (liquid hourly space velocity) of a raw material on hydrocracking with the use of Ga-MFI/SiO2 catalyst (Si/T=17.3) according to the second embodiment;
[Figure 9] Figure 9 includes a chart showing a change of results of a stability evaluation test with time, i.e., results of test for evaluation of the stability of hydrocracking with the use of Ga-MFI/SiO2 catalyst (Si/T=17.3) according to the second embodiment; and
[Figure 10] Figure 10 includes a chart showing the effect of LHSV of a raw material on hydrocracking with the use of Ga-MFI/SiO2 catalyst (Si/T=10.1) according to the second embodiment.

### DESCRIPTION OF THE PREFERRED EMBODIMENT(S)

### <First Embodiment>

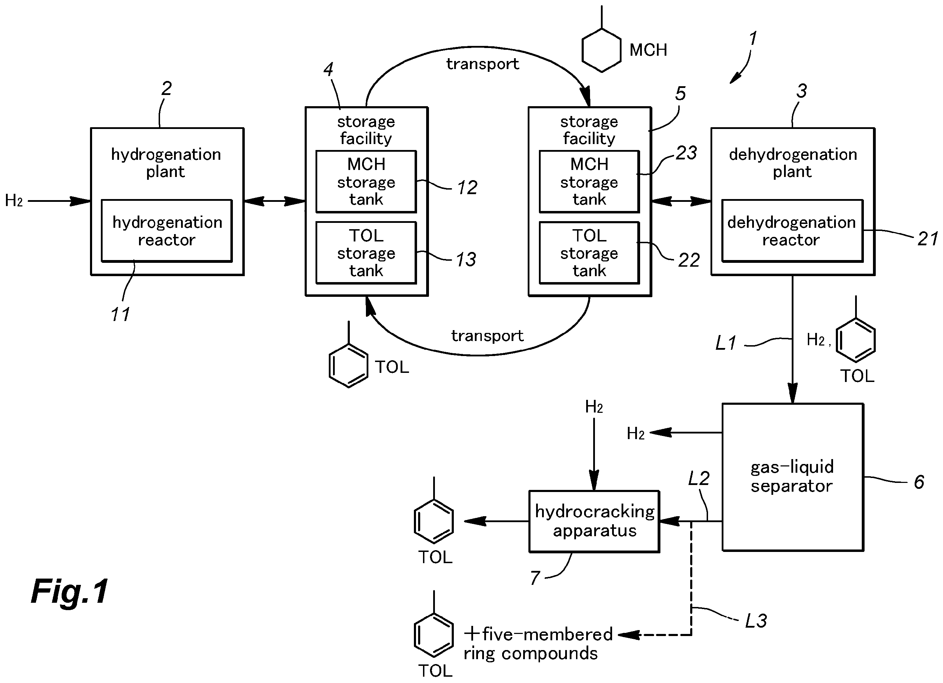

A method for hydrocracking a cyclic compound having a five-membered ring structure using a zeolite composite catalyst used for hydrocracking a cyclic compound having a five-membered ring structure (herein also referred to as "five-membered ring compound(s)") according to a first embodiment of the present invention will be described with reference to the appended drawings. In embodiments described below, a zeolite composite catalyst and a method for hydrocracking a compound using the same according to the present invention are embodied in a hydrogen storage and transportation system in which an organic chemical hydride method is carried out, as shown in Figure 1.

The hydrogen storage and transportation system 1 includes a hydrogenation plant 2 that generates saturated cyclic compounds as organic hydrides by adding hydrogen to aromatic compounds, and a dehydrogenation plant 3 that generates hydrogen and aromatic compounds by dehydrogenating the saturated cyclic compounds. In the following example, a saturated cyclic compound to be used is methylcyclohexane (hereafter also referred to as "MCH" as necessary) or cyclohexane (hereafter also referred to as "CH" as necessary), and a corresponding aromatic compound to be used is toluene (hereafter also referred to as "TO" as necessary) or benzene (hereafter also referred to as "BZ" as necessary).

The hydrogenation plant 2 is provided with a hydrogenation reactor 11 that produces methylcyclohexane by causing a hydrogenation reaction to add hydrogen (H₂) to toluene, using a known method. The system includes a storage facility 4 associated with the hydrogenation plant 2. The storage facility 4 includes an MCH storage tank 12 for storing the methylcyclohexane produced by the hydrogenation reactor 11. The storage facility 4 also includes a TOL storage tank 13 for storing toluene transported from the dehydrogenation plant 3 (in a storage facility 5).

The dehydrogenation plant 3 is provided with a dehydrogenation reactor 21 that produces hydrogen and toluene by causing a dehydrogenation reaction of methylcyclohexane, using a known method. The system includes the storage facility 5 associated with the dehydrogenation plant 3. The storage facility 5 includes a TOL storage tank 22 for storing the toluene produced by the dehydrogenation reactor 21. The storage facility 5 also includes an MCH storage tank 23 for storing the methylcyclohexane transported from the hydrogenation plant 2 (in the storage facility 4).

In the hydrogen storage and transportation system 1, the methylcyclohexane stored in the MCH storage tank 12 is transported to the dehydrogenation plant 3 using a known means for transportation (such as ship, vehicle, or pipeline) and then stored in the MCH storage tank 23. The methylcyclohexane stored in the MCH storage tank 23 is used in a dehydrogenation reaction in the dehydrogenation reactor 21. The toluene stored in the toluene storage tank 22 is transported to the hydrogenation plant 2 using a known means for transportation and stored in the toluene storage tank 13. The toluene stored in the toluene storage tank 13 is used in the hydrogenation reaction in the hydrogenation reactor 11. In this way, the methylcyclohexane and toluene are repeatedly used as containers for hydrogen (hydrogen carriers).

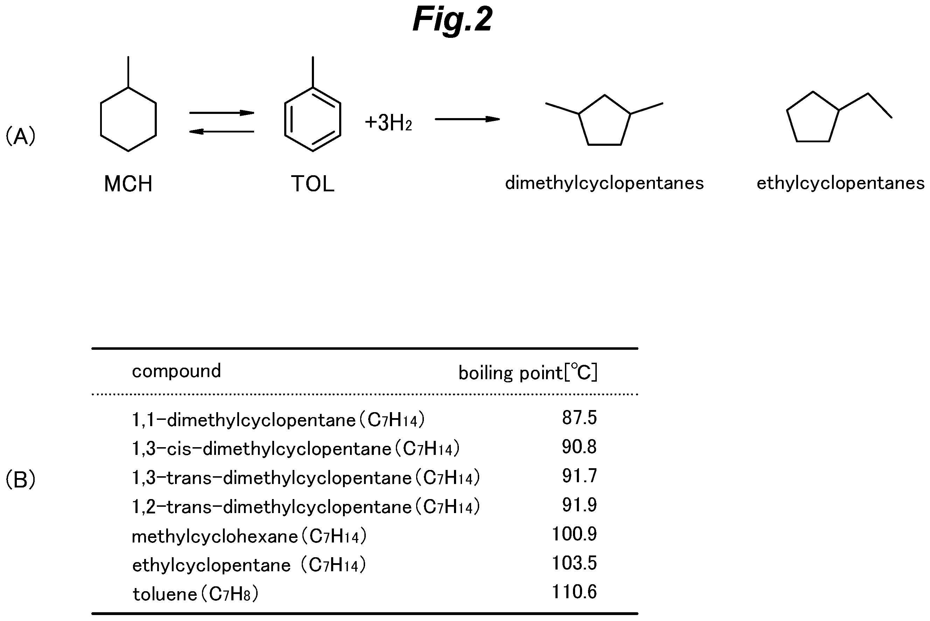

As shown in Figure 2(A), the dehydrogenation reaction in the dehydrogenation reactor 21 generates, in addition to hydrogen and toluene, by-products containing five-membered ring compounds, as reaction products. Such five-membered ring compounds include dimethylcyclopentanes (e.g., 1,1-dimethylcyclopentane, 1,3-cis-dimethylcyclopentane, 1,3-trans-dimethylcyclopentane, and 1,2-trans-dimethylcyclopentane) and ethylcyclopentane. The boiling points of the five-membered ring compounds are relatively close to those of methylcyclohexane and toluene, which are used as hydrogen carriers, as shown in Figure 2(B), which makes it difficult to separate the five-membered ring compounds from the hydrogen carrier through distillation.

The five-membered ring compounds produced in dehydrogenation reactor 21 are mixed with toluene and stored in the TOL storage tank 22, and then transported to the hydrogenation plant 2 via the TOL storage tank 13. Thus, the methylcyclohexane produced in the hydrogenation plant 2 may contain the five-membered ring compounds derived from the above-described dehydrogenation reaction as impurities. The five-membered ring compounds mixed with methylcyclohexane, are transported to the dehydrogenation plant 3 again, and thus circulate within the hydrogen storage and transportation system 1. The five-membered ring compounds can be produced not only by the dehydrogenation reaction in the dehydrogenation reactor 21, but also by the hydrogenation reaction in the hydrogenation reactor 11.

Hence, the hydrogen storage and transportation system 1 further includes a gas-liquid separator 6 that separates the reaction products generated in the dehydrogenation reactor 21 in the dehydrogenation plant 3 into gas and liquid, and a hydrocracking apparatus 7 that causes a hydrocracking reaction in the liquid separated by the gas-liquid separator 6. **In** the system shown in Figure 1, the gas-liquid separator 6 and hydrocracking apparatus 7 are separately provided from the dehydrogenation plant 3, but may be provided as parts of the dehydrogenation plant 3.

The gas-liquid separator 6 is connected to a reaction product discharge line L1 of the dehydrogenation reactor 21. The gas-liquid separator 6 separates products of the reaction in the dehydrogenation reactor 21 into a gas phase (gas) consisting mainly of hydrogen and a liquid phase (liquid) consisting mainly of toluene. The liquid contains five-membered ring compounds as impurities of toluene. The liquid containing toluene and the five-membered ring compounds is supplied to the hydrocracking apparatus 7. The gas containing hydrogen is supplied to the hydrogen storage tank (not shown). The gas-liquid separator 6 may be provided with a cooler for cooling the products of the dehydrogenation reaction. The gas-liquid separator 6 is provided with a discharge line L2 for discharging the liquid containing toluene and the five-membered ring compounds.

The hydrocracking apparatus 7 is connected to the discharge line L2 of the gas-liquid separator 6. The hydrocracking apparatus 7 uses a zeolite composite catalyst (hereinafter referred to as a "hydrogenolysis catalyst") to selectively hydrocrack the five-membered ring compounds that have been mixed into toluene as impurities to convert the five-membered ring compounds into linear light hydrocarbons. In the hydrocracking apparatus 7, the five-membered ring compounds mixed in toluene are broken down into lighter chain-like hydrocarbons (such as C1-C4 alkanes, pentane, hexane, and heptane). These hydrocarbons can be removed from toluene by distillation or any other known suitable process. The toluene from which the five-membered ring compound has been removed (to be decreased) is mixed again with the toluene stored in the TOL storage tank 22.

In the hydrogen storage and transportation system 1, a portion of the liquid (including toluene and five-membered ring compounds) separated by the gas-liquid separator 6 can be excluded from the subject of hydrocracking, i.e., not being supplied to the hydrocracking apparatus 7. For example, in the hydrogen storage and transportation system 1, a branch line L3 is provided to branch from the discharge line L2, and at least a part (0-100%) of the liquid separated by the gas-liquid separator 6 may be supplied to the branch line L3 by operating a valve (not shown) or any other suitable device. The downstream end of the branch line L3 is connected to the TOL storage tank 22, and the liquid excluded from the subject of hydrocracking is stored in the TOL storage tank 22.

Determination of the amount (percentage) of liquid excluded from the subject of hydrocracking can be made based on, for example, the concentration of the five-membered ring compounds mixed in toluene, the performance of the hydrocracking apparatus 7 (such as the five-membered ring compound decrease rate, or the hydrogen carrier loss rate as described later), and the amount of hydrogen carrier required by the system. For example, when the concentration of five-membered ring compounds mixed in toluene is close to (or below) a prescribed target value, the proportion of liquid supplied from the gas-liquid separator 6 to the hydrocracking apparatus 7 can be set low (or to zero). When the concentration of the five-membered ring compounds mixed in toluene is higher than the target value, the proportion of liquid supplied from the gas-liquid separator 6 to the hydrocracking apparatus 7 can be set higher (or to 100%).

The subject to be processed by the hydrocracking apparatus 7 of the first embodiment may be toluene (containing five-membered ring compounds as impurities) that has been once stored in the TOL storage tank 22 (or the TOL storage tank 13). In this case, the toluene from which the five-membered ring compounds have been removed by the hydrocracking apparatus 7 is returned to the TOL storage tank 22 (or the TOL storage tank 13).

For example, a fixed-bed circulating reactor is used as the hydrocracking apparatus 7. The hydrocracking apparatus 7 includes a temperature controller (e.g., an electric heater or a heat exchanger using a heat medium). Preferably, when the hydrocracking reaction occurs in the hydrocracking apparatus 7, the temperature of a catalyst layer comprising the hydrogenolysis catalyst is maintained within a range of 200°C to 450°C by the temperature controller. Examples of reaction raw materials used in the hydrocracking reaction may include a mixture of toluene and five-membered ring compounds, along with 5-90 mol% hydrogen.

Examples of the hydrogenolysis catalyst used in the hydrocracking apparatus 7 may include a zeolite catalyst (excluding silicalite) containing a zeolite with an MFI structure as a framework structure (i.e., zeolite with a framework type code MFI). In particular, an example of preferable one is a zeolite composite catalyst comprising zeolite that contains at least one of aluminum, gallium, and iron and has the MFI-type structure, as well as silica (silicon dioxide) as a binder. The binder is a binding agent used to give the zeolite into a predetermined shape (e.g., cylindrical).

When the zeolite contains gallium, the zeolite with an MFI structure used as a hydrogenolysis catalyst, preferably has an acid density of 5 to 200 where the acid density is determined as a ratio of an amount of silicon to an amount of gallium, the amounts being expressed in moles. When the acid density is less than 5, it becomes difficult to prepare the zeolite with an MFI structure as a hydrogenolysis catalyst. When the acid density exceeds 200, the effect of the decrease in five-membered ring compounds (the five-membered ring compound decrease rate as described later) becomes lower, making it difficult to ensure the necessary catalytic performance. The same applies to zeolites with an MFI structure that contain metals other than gallium (at least one of iron and aluminum).

When the zeolite with an MFI structure contains iron and aluminum, the zeolite preferably has an acid density of 5 to 200 where the acid density is determined as a ratio of an amount of silicon to a sum of amounts of iron and aluminum, and also has an iron ratio of 0.1 to 0.9 where the iron ratio is a ratio of an amount of iron to a sum of amounts of iron and aluminum, all the amounts being expressed in moles. This iron ratio allows the acid strength of an acid site to be properly adjusted.

When the zeolite with an MFI structure contains aluminum, the zeolite preferably has an acid density of 5 to 200 where the acid density is determined as a ratio of an amount of silicon to an amount of aluminum, the amounts being expressed in moles.

The organic hydrides used in the hydrogen storage and transportation system 1 can be any organic compound that reversibly releases hydrogen through a catalytic reaction, and is not limited to the methylcyclohexane as described above. The organic hydrides that can be used in the hydrogen storage and transportation system 1 may include a monocyclic hydrogenated aromatic compound such as methylcyclohexane or cyclohexane, a bicyclic hydrogenated aromatic compound such as tetralin, decalin, or methyl decalin, and a tricyclic hydrogenated aromatic compound such as tetradecahydroanthracene either alone or in combination. When the organic hydride is changed, another aromatic compound corresponding thereto produced by a dehydrogenation reaction is used in place of toluene.

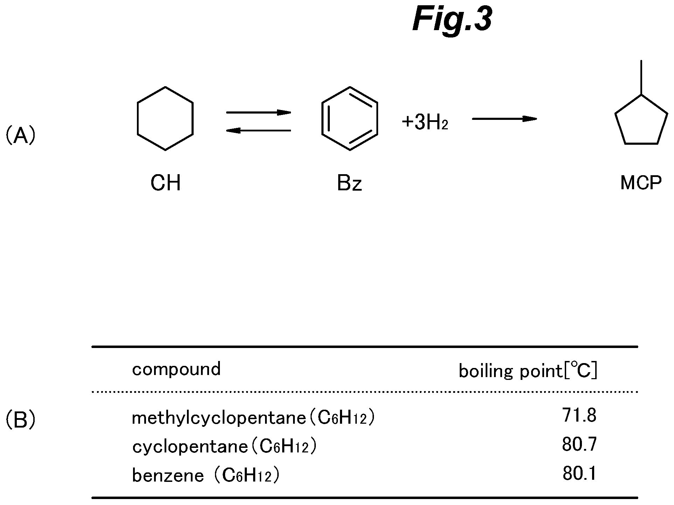

Figure 3 is a diagram of examples other than methylcyclohexane, showing the relationship between the by-products of the cyclohexane dehydrogenation reaction and their boiling points. The cyclohexane dehydrogenation reaction produces, in addition to hydrogen and benzene, by-products including methylcyclopentane, a five-membered ring compound as reaction products (Figure 3(A)). The boiling point of methylcyclopentane is relatively close to that of cyclohexane and benzene, which are used as hydrogen carriers (Figure 3(B)), and, as in the case of methylcyclohexane dehydrogenation (Figure 2(B)), separation of cyclohexane and benzene from the hydrogen carrier by distillation is not easy.

The hydrogenolysis catalysts of the first embodiment and methods for hydrocracking a five-membered ring compound using the catalyst of preferred embodiments will be described with reference to Examples 1-11 and Comparative Example 1.

The above-described hydrocracking apparatus 7 is configured to be used to hydrocrack the five-membered ring compounds produced as a by-product together with the main product (in this case, toluene) of the dehydrogenation reaction in the dehydrogenation reactor 21 using a hydrogenolysis catalyst. A reaction raw material (i.e. a material used in the hydrocracking reaction) in the hydrocracking apparatus 7 is a mixture of toluene or other compounds produced by the reaction of dehydrogenation of methylcyclohexane, and the five-membered ring compounds.

In Examples 1-6, 9-11 and Comparative Example 1, a mixture of a solution (hereinafter referred to as "toluene solution") with the composition shown in Table 1 and a predetermined amount of hydrogen (27 mol% hydrogen) was used as a reaction raw material for evaluating the performance of each of the catalyst. However, in Examples 9-11, the amount of hydrogen in the reaction raw material was varied. The toluene solution was a liquid-phase product (reaction product from which gas has been separated) after the dehydrogenation reaction. The toluene solution contained a hydrogen carrier including more than 96 wt% toluene and about 2 wt% unreacted methylcyclohexane and also contains about 0.5 to 1.0 wt% five-membered ring compounds (see the components surrounded by a dotted line in Table 1) as impurities.

In Examples 7 and 8, a mixture of a solution (hereinafter referred to as "benzene solution") with the composition shown in Table 2 and a predetermined amount of hydrogen (27 mol% hydrogen) was used as a reaction raw material for evaluating the performance of each of the catalysts. The benzene solution was a liquid-phase product (reaction product from which gas has been separated) after cyclohexane dehydrogenation reaction. The benzene solution contained a hydrogen carrier including more than 97 wt% benzene and about 2 wt% unreacted cyclohexane, and also contains about 0.5 wt% five-membered ring compounds (see the amount of methylcyclopentane surrounded by a dotted line in Table 2) as an impurity.

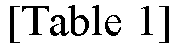

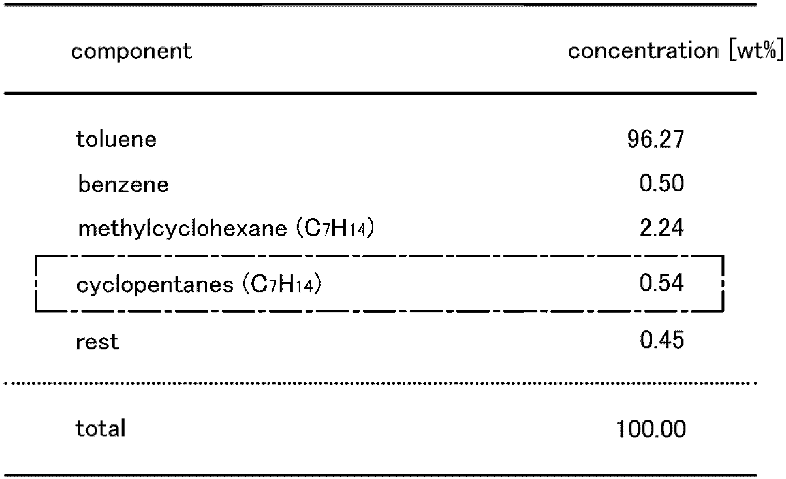

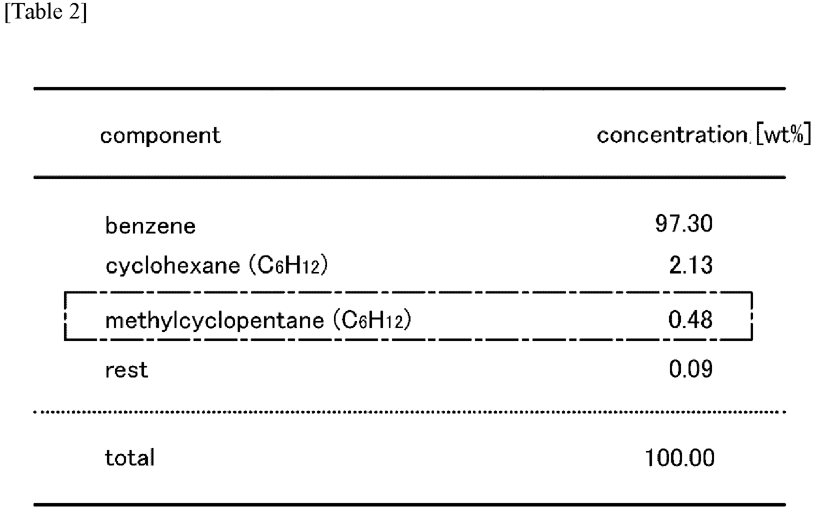

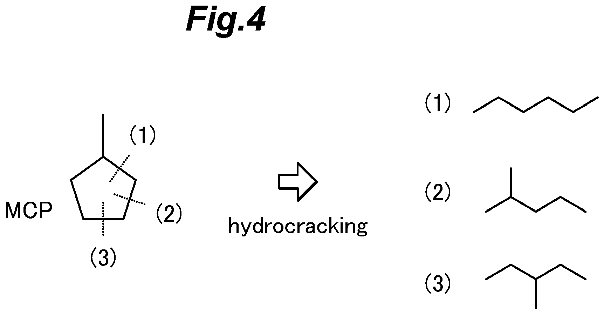

Figure 4 shows an example of the hydrocracking of methylcyclopentane (also referred to as "MCP"), which is a model compound for five-membered ring compound. The hydrocracking of MCP can cause the ring to break at positions (1) to (3), thereby producing resulting chain-type compounds (such as hexane, 2-methylpentane, and 3-methylpentane). In addition, these chain-type compounds can be further broken down to produce C1-C5 alkanes. Unlike five-membered ring compounds, the products of hydrocracking reactions can be easily separated from benzene or toluene using distillation or other known processes. Similarly, the products of hydrocracking reactions of ethylcyclopentane and other five-membered ring compounds can also be easily separated from toluene.

Table 3 shows a list of the various catalyst samples used in Examples 1-11 and Comparative Example 1. As will be described in detail later, Examples 1 and 7 used a zeolite composite catalyst (hereinafter referred to as "Al-MFI/SiO₂ catalyst") containing a zeolite with an MFI structure that includes aluminum (Al) and silica (SiO₂) as a binder. The Al-MFI/SiO₂ catalyst has an acid site(s) derived from aluminum. The silica used as a binder can function to coat and deactivate the acid site(s) on the outer surface of the zeolite. This inhibits reactions of aromatic hydrocarbons (in this case, toluene) and saturated cyclic compounds (in this case, methylcyclohexane) at the acid site(s) of the zeolite. Example 2 used a zeolite composite catalyst (hereinafter referred to as the "Ga-MFI/SiO₂ catalyst") containing a zeolite with an MFI structure that includes gallium (Ga) and silica as a binder. The Ga-MFI/SiO2 catalyst has an acid site(s) derived from gallium. Examples 3-6 and 8-11 used a zeolite composite catalyst (hereinafter referred to as the "Fe-Al-MFI/SiO2 catalyst") containing a zeolite with an MFI structure that contained iron (Fe) and aluminum (Al), as well as silica as a binder. This catalyst has acid sites derived from iron and aluminum. In addition, Fe-Al-MFI/SiO₂ catalysts used in Examples 4-6 and 8 were similar zeolite composite catalysts to those used in Examples 3 and 9-11, respectively, but with different compositions (different ratios of silicon (Si), aluminum (Al), and iron (Fe)). Comparative Example 1 used a zeolite composite catalyst (hereinafter referred to as the "Al-beta/SiO₂ catalyst") containing a zeolite with a BETA structure that included aluminum (Al) and silica (SiO₂) as a binder. The catalyst samples used in Examples 1-11 and Comparative Example 1 were configured to have the approximately same amount of acid sites.

**[Table 3]**

| | catalyst | cata lyst compos sition [mol /mol] | | | | binder | zeolite/binder MR [wt%/wt%] |
|---|---|---|---|---|---|---|---|
| | | Si/T | Al/T | Ga/T | Fe/T | | |
| C. Ex. 1 | Al-BETA/SiO₂ | 20.0 | 1.00 | 0.00 | 0.00 | SiO₂ | 65/35 |
| Ex. 1, 7 | Al-MFI/SiO2 | 21.3 | 1.00 | 0.00 | 0.00 | SiO₂ | 65/35 |
| Ex. 2 | Ga-MFI/SiO₂ | 17.3 | 0.06 | 0.94 | 0.00 | SiO₂ | 65/35 |
| Ex. 3, 9-11 | Fe-Al-MFI/SiO₂ | 19.4 | 0.72 | 0.00 | 0.28 | SiO₂ | 65/35 |
| Ex. 4 | Fe-Al-MFI/SiO₂ | 18.9 | 0.60 | 0.00 | 0.40 | SiO₂ | 65/35 |
| Ex. 5, 8 | Fe-Al-MFI/SiO₂ | 18.9 | 0.54 | 0.00 | 0.46 | SiO₂ | 65/35 |
| Ex. 6 | Fe-Al-MFI/SiO₂ | 17.0 | 0.33 | 0.00 | 0.67 | SiO₂ | 65/35 |

In Table 3, "T", which is used to indicate the composition (in moles) of the catalyst, represents the amount (in moles) of aluminum, iron + aluminum, or gallium + aluminum in a zeolite composite catalyst. For example, in the Al-MFI/SiO₂ catalysts of Examples 1 and 7, the "Si/T" represents the ratio of Si (silicon) to T (aluminum), and the "Al/T" represents the ratio of Al (aluminum) to T (aluminum). In Examples 1 and 7, "T" is the amount of Al, and thus Al/T = 1. Amorphous silica powder (AEROSIL (Registered Trademark) 380, Nihon Aerosil Co., Ltd., BET specific surface area 380 m²/g) was used as the binder (SiO₂). The mixing ratio of zeolite and a binder was 65/35 for all the catalysts.

Table 4 shows a list of conditions of a performance evaluation test for each catalyst sample used in Examples 1-6 and Comparative Example 1, and Table 5 shows a list of conditions of a performance evaluation test for each catalyst sample used in Examples 7 and 8. As will be described in detail later, in Examples 1-6 and Comparative Example 1, the catalytic performance of each sample was evaluated using a toluene solution with the composition shown in Table 1 as the raw material. In Examples 7 and 8, the catalytic performance of each sample was evaluated using a benzene solution with the composition shown in Table 2 as the raw material.

**[Table 4]**

| | reaction temperature [°C] | reaction pressure [MPa] | LHSV of raw material solution [h⁻¹] | flow rate (concentration) of hydrogen [L/min (mol%)] |
|---|---|---|---|---|
| C. Ex. 1 | 350 | 0.30 | 6.0 | 0.02 (27) |
| | 375 | 0.30 | 6.0 | 0.02 (27) |
| | 400 | 0.30 | 6.0 | 0.02 (27) |
| Ex. 1 | 350 | 0.30 | 6.0 | 0.02 (27) |
| | 375 | 0.30 | 6.0 | 0.02 (27) |
| | 400 | 0.30 | 6.0 | 0.02 (27) |
| Ex. 2 | 350 | 0.30 | 6.0 | 0.02 (27) |
| | 375 | 0.30 | 6.0 | 0.02 (27) |
| | 400 | 0.30 | 6.0 | 0.02 (27) |
| Ex. 3 | 350 | 0.30 | 6.0 | 0.02 (27) |
| | 375 | 0.30 | 6.0 | 0.02 (27) |
| | 400 | 0.30 | 6.0 | 0.02 (27) |
| Ex. 4 | 350 | 0.30 | 6.0 | 0.02 (27) |
| | 375 | 0.30 | 6.0 | 0.02 (27) |
| | 400 | 0.30 | 6.0 | 0.02 (27) |
| Ex_ 5 | 350 | 0.30 | 6.0 | 0.02 (27) |
| | 375 | 0.30 | 6.0 | 0.02 (27) |
| | 400 | 0.30 | 6.0 | 0.02 (27) |
| Ex. 6 | 350 | 0.30 | 6.0 | 0.02 (27) |
| | 375 | 0.30 | 6.0 | 0.02 (27) |
| | 400 | 0.30 | 6.0 | 0.02 (27) |

**[Table 5]**

| | reaction temperature [°C] | reaction pressure [MPa] | LHSV of raw material solution [h⁻¹] | flow rate (concentration) of hydrogen [L/min (mol%)] |
|---|---|---|---|---|
| Ex. 7 | 350 | 0.30 | 6.0 | 0.02 (27) |
| | 400 | 0.30 | 6.0 | 0.02 (27) |
| Ex. 8 | 350 | 0.30 | 6.0 | 0.02 (27) |
| | 400 | 0.30 | 6.0 | 0.02 (27) |

### (Example 1)

### <Method for preparing Al-MFI/SiO₂ catalyst>

### Method for synthesizing Al-MFI zeolite

A solution comprising 58.9g colloidal silica (30.6 wt% SiO₂, 0.4 wt% Na₂O, 69.0 wt% H₂O), and 2.99g sodium hydroxide was prepared as Solution A, and a solution comprising 3.80g aluminum sulfate, n-hydrate, 9.29g tetrapropylammonium bromide, and 186.27g purified water was prepared as Solution B. After the solutions A and B were gradually mixed while being stirred at room temperature, the mixture was stirred vigorously in a mixer for a further 15 minutes. After this mixed solution was kept at 60°C overnight, hydrothermal synthesis was carried out under autoclave conditions at 150°C, 72 hours, and 900 rpm under naturally occurring pressure. After being cooled, the product was thoroughly washed with purified water. Then, the product was dried at 120°C for 3 hours and calcined at 550°C for 3 hours in air circulation, which resulted in the synthesis of pr powdered Na-type MFI zeolite containing Al (hereafter referred to as Al-MFI zeolite). Through X-ray fluorescence analysis, the elemental composition (in moles) of the zeolite was determined to be Si/Al = 21.3 (Table 3).

### Method for preparing Al-MFI/SiO₂ catalyst

After the powdered Na-type Al-MFI zeolite synthesized according to the above-described process was mixed with silica powder (AEROSIL (Registered Trademark) 380, Japan Aerosil Co., Ltd., BET specific surface area 380 m²/g) as a binder, and a certain amount of starch as a forming agent, the mixture was kneaded with the addition of a proper amount of a sodium hydroxide aqueous solution (4.5 wt% NaOH), which produced a cubical zeolite/silica mixture. The resulting product was then processed into a cylindrical shape (1.0 mm φ) using an extruder, dried at 120°C for 3 hours, and calcined at 550°C for 3 hours under air circulation, which produced an Al-MFI zeolite/silica composite. The resulting composite was subjected to ion exchange with a 2.2 mol/L aqueous ammonium nitrate solution under boiling reflux, followed by four water washes (each ion exchange was carried out for 2 hours, and the ammonium nitrate solution was replaced with a fresh 2.2 mol/L aqueous ammonium nitrate solution each time). Then, the resulting product was dried at 120°C for 3 hours and calcined at 550°C for 3 hours under air circulation to prepare a proton-type Al-MFI zeolite/silica composite catalyst (hereafter referred to as "Al-MFI/SiO₂ catalyst"). The composition (in weight) of this catalyst was determined to be 65/35 [wt%/wt%] Al-MFI/SiO₂ by X-ray fluorescence analysis (Table 3).

### <Method of performance evaluation test for Al-MFI/SiO₂ catalyst>

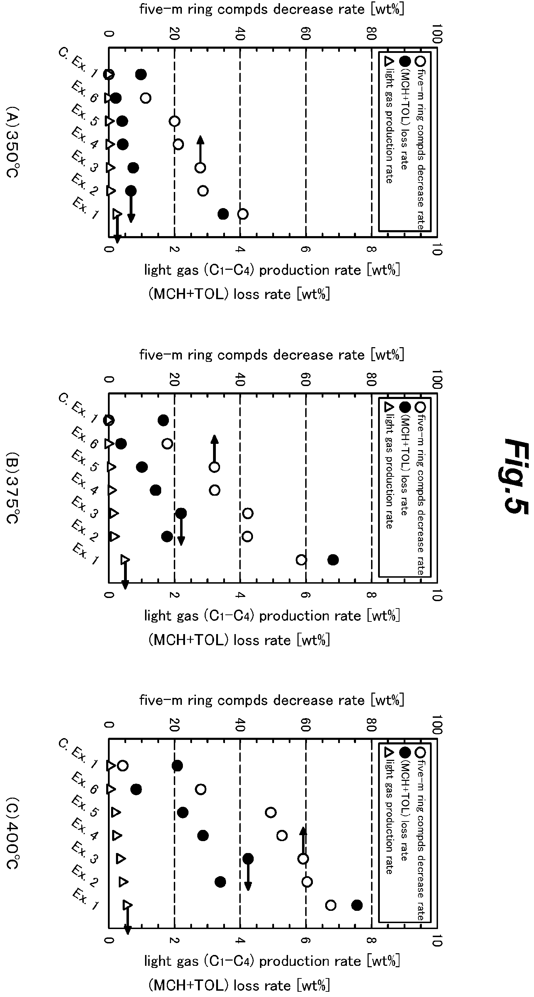

The performance evaluation test for the above-described Al-MFI/SiO₂ catalyst was conducted using the following procedure. The Al-MFI/SiO₂ catalyst was packed into a fixed-bed, circulating-type reactor as a 2.32 mL catalyst bed. The reaction raw material (Table 1) was supplied into the reactor at a flow rate of 0.235 mL/min (LHSV (liquid hourly space velocity) 6 h⁻¹) together with hydrogen at a flow rate of 0.02 L/min (27 mol%), and the hydrocracking reaction of the reaction raw material was caused at a pressure of 0.30 MPa and average temperatures of the catalyst bed (i.e., reaction temperatures) of 350°C, 375°C, and 400°C for a predetermined time (Table 4). Two hours after the hydrocracking reaction started at each catalyst bed temperature, the gas-phase and liquid-phase products were sampled. Then, the five-membered ring compound decrease rate (wt%), the MCH+TOL (hydrogen carrier) loss rate, and the light gas production rate (wt%), i.e., the rate of production of light gas components (C₁-C₄) were determined based on the results of FID-GC (flame ionization detector gas chromatograph) analysis, and these were used as indicators of catalyst performance (The results are shown in Figure 5 and Table 6 as described later).

### (Example 2)

### <Method for preparing Ga-MFI/SiO2 catalyst>

### Method for synthesizing Ga-MFI zeolite

A powdered Na-type MFI zeolite containing Ga (hereinafter referred to as Ga-MFI zeolite) was synthesized in the same manner as Example 1 except that a solution comprising 4.57g gallium nitrate pentahydrate, 9.29g tetrapropylammonium bromide, and 186.52g purified water was used as the Solution B. Through X-ray fluorescence analysis, the elemental composition (in moles) of the zeolite was determined to be Si/Ga = 17.3 (Table 3).

### Method for preparing Ga-MFI/SiO₂ catalyst

The powdered Na-type Ga-MFI zeolite synthesized according to the above-described process was used to prepare a proton-type Ga-MFI zeolite/silica composite catalyst (hereafter referred to as "Ga-MFI/SiO₂ catalyst") in the same manner as Example 1. The composition (in weight) of this catalyst was determined to be 65/35 [wt%/wt%] Ga-MFI/SiO₂ by X-ray fluorescence analysis (Table 3).

### <Method of performance evaluation test for Ga-MFI/SiO₂ catalyst>

The performance evaluation test for the above-described Ga-MFI/SiO₂ catalyst was conducted in the same manner as Example 1 (Table 4). The results are shown in Figure 5 and Table 6 as described later.

### (Example 3)

### <Method for preparing Fe-Al-MFI/SiO₂ catalyst>

### Method for synthesizing Fe-Al-MFI zeolite

A powdered Na-type MFI zeolite containing Fe and Al (hereinafter referred to as Fe-Al-MFI zeolite) was synthesized in the same manner as Example 1 except that a solution comprising 3.04g aluminum sulfate, n-hydrate; 0.98g iron sulfate, 9-hydrate; 9.29g tetrapropylammonium bromide; and 186.22g purified water was used as the Solution B. Through X-ray fluorescence analysis, the elemental composition (in moles) of the zeolite was determined to be Si/(Fe + Al) = 19.4, Al/(Fe + Al) = 0.72, Fe/(Fe + Al) = 0.28 (Table 3).

### Method for preparing Fe-Al- MFI/SiO₂ catalyst

The powdered Na-type Fe-Al-MFI zeolite synthesized according to the above-described process was used to prepare a proton-type Fe-Al-MFI zeolite/silica composite catalyst (hereafter referred to as "Fe-Al-MFI/SiO₂ catalyst") in the same manner as Example 1. The composition (in weight) of this catalyst was determined to be 65/35 [wt%/wt%] Fe-Al-MFI/SiO₂ by X-ray fluorescence analysis (Table 3).

### <Method of performance evaluation test for Fe-Al-MFI/SiO₂ catalyst>

The performance evaluation test for the above-described Fe-Al-MFI/SiO₂ catalyst was conducted in the same manner as Example 1 (Table 4). The results are shown in Figure 5 and Table 6 as described later.

### (Example 4)

### <Method for preparing Fe-Al-MFI/SiO₂ catalyst>

### Method for synthesizing Fe-Al-MFI zeolite

A powdered Na-type MFI zeolite containing Fe and Al (hereinafter referred to as Fe-Al-MFI zeolite) was synthesized in the same manner as Example 1 except that a solution comprising 2.66g aluminum sulfate, n-hydrate; 1.47g iron sulfate, 9-hydrate; 9.29g tetrapropylammonium bromide; and 186.20g purified water was used as the Solution B. Through X-ray fluorescence analysis, the elemental composition (in moles) of the zeolite was determined to be Si/(Fe + Al) = 18.9, Al/(Fe + Al) = 0.60, Fe/(Fe + Al) = 0.40 (Table 3).

### Method for preparing Fe-Al- MFI/SiO₂ catalyst

The powdered Na-type Fe-Al-MFI zeolite synthesized according to the above-described process was used to prepare a proton-type Fe-Al-MFI zeolite/silica composite catalyst (hereafter referred to as "Fe-Al-MFI/SiO₂ catalyst") in the same manner as Example 1. The composition (in weight) of this catalyst was determined to be 65/35 [wt%/wt%] Fe-Al-MFI/SiO₂ by X-ray fluorescence analysis (Table 3).

### <Method of performance evaluation test for Fe-Al-MFI/SiO₂ catalyst>

The performance evaluation test for the above-described Fe-Al-MFI/SiO₂ catalyst was conducted in the same manner as Example 1 (Table 4). The results are shown in Figure 5 and Table 6 as described later.

### (Example 5)

### <Method for preparing Fe-Al-MFI/SiO₂ catalyst>

### Method for synthesizing Fe-Al-MFI zeolite

A powdered Na-type MFI zeolite containing Fe and Al (hereinafter referred to as Fe-Al-MFI zeolite) was synthesized in the same manner as Example 1 except that a solution comprising 2.29g aluminum sulfate, n-hydrate; 1.94g iron sulfate, 9-hydrate; 9.29g tetrapropylammonium bromide; and 186.18g purified water was used as the Solution B. Through X-ray fluorescence analysis, the elemental composition (in moles) of the zeolite was determined to be Si/(Fe + Al) = 18.9, Al/(Fe + Al) = 0.54, Fe/(Fe + Al) = 0.46 (Table 3).

### Method for preparing Fe-Al- MFI/SiO₂ catalyst

The powdered Na-type Fe-Al-MFI zeolite synthesized according to the above-described process was used to prepare a proton-type Fe-Al-MFI zeolite/silica composite catalyst (hereafter referred to as "Fe-Al-MFI/SiO₂ catalyst") in the same manner as Example 1. The composition (in weight) of this catalyst was determined to be 65/35 [wt%/wt%] Fe-Al-MFI/SiO₂ by X-ray fluorescence analysis (Table 3).

### <Method of performance evaluation test for Fe-Al-MFI/SiO₂ catalyst>

The performance evaluation test for the above-described Fe-Al-MFI/SiO₂ catalyst was conducted in the same manner as Example 1 (Table 4). The results are shown in Figure 5 and Table 6 as described later.

### (Example 6)

### <Method for preparing Fe-Al-MFI/SiO₂ catalyst>

### Method for synthesizing Fe-Al-MFI zeolite

A powdered Na-type MFI zeolite containing Fe and Al (hereinafter referred to as Fe-Al-MFI zeolite) was synthesized in the same manner as Example 1 except that a solution comprising 1.52g aluminum sulfate, n-hydrate; 2.94g iron sulfate, 9-hydrate; 9.29g tetrapropylammonium bromide; and 186.14g purified water was used as the Solution B. Through X-ray fluorescence analysis, the elemental composition (in moles) of the zeolite was determined to be Si/(Fe + Al) = 17.0, Al/(Fe + Al) = 0.33, Fe/(Fe + Al) = 0.67 (Table 3).

### Method for preparing Fe-Al- MFI/SiO₂ catalyst

The powdered Na-type Fe-Al-MFI zeolite synthesized according to the above-described process was used to prepare a proton-type Fe-Al-MFI zeolite/silica composite catalyst (hereafter referred to as "Fe-Al-MFI/SiO₂ catalyst") in the same manner as Example 1. The composition (in weight) of this catalyst was determined to be 65/35 [wt%/wt%] Fe-Al-MFI/SiO₂ by X-ray fluorescence analysis (Table 3).

### <Method of performance evaluation test for Fe-Al-MFI/SiO₂ catalyst>

The performance evaluation test for the above-described Fe-Al-MFI/SiO₂ catalyst was conducted in the same manner as Example 1 (Table 4). The results are shown in Figure 5 and Table 6 as described later.

### (Example 7)

### <Method for preparing Al-MFI/SiO₂ catalyst>

### Method for synthesizing Al-MFI zeolite

A powdered Na-type MFI zeolite containing Al (i.e., Al-MFI zeolite) was synthesized in the same manner as Example 1. Through X-ray fluorescence analysis, the elemental composition (in moles) of the zeolite was determined to be Si/Ga = 21.3 (Table 3).

### Method for preparing Al-MFI/SiO₂ catalyst

The powdered Na-type Al-MFI zeolite synthesized according to the above-described process was used to prepare a proton-type Al-MFI zeolite/silica composite catalyst (i.e., Al-MFI/SiO₂ catalyst) in the same manner as Example 1. The composition (in weight) of this catalyst was determined to be 65/35 [wt%/wt%] Al-MFI/SiO₂ by X-ray fluorescence analysis (Table 3).

### <Method of performance evaluation test for Al-MFI/SiO₂ catalyst>

The performance evaluation test for the above-described Al-MFI/SiO₂ catalyst was conducted using the following procedure. The Al-MFI/SiO₂ catalyst was packed into a fixed-bed, circulating-type reactor as a 2.32mL catalyst bed. The reaction raw material (Table 2) was supplied into the reactor at a flow rate of 0.235 mL/min (LHSV (liquid hourly space velocity) 6 h⁻¹) together with hydrogen at a flow rate of 0.02 L/min (27 mol%), and the hydrocracking reaction of the reaction raw material was caused at a pressure of 0.30 MPa and average temperatures of the catalyst bed (i.e., reaction temperatures) of 350°C, 375°C, and 400°C for a predetermined time (Table 5). Two hours after the hydrocracking reaction started at each catalyst bed temperature, the gas-phase and liquid-phase products were sampled. Then, the five-membered ring compound (MCP) decrease rate (wt%), the cyclohexane + benzene (hydrogen carrier) loss rate, and the light gas production rate (wt%), i.e., the rate of production of light gas components (C₁-C₄) were determined based on the results of FID-GC (flame ionization detector gas chromatograph) analysis, and these were used as indicators of catalyst performance. (The results are shown in Table 7 as described later.)

### (Example 8)

### <Method for preparing Fe-Al-MFI/SiO₂ catalyst>

### Method for synthesizing Fe-Al-MFI zeolite

A powdered Na-type MFI zeolite containing Fe and Al (i.e., Fe-Al-MFI zeolite) was synthesized in the same manner as Example 5. Through X-ray fluorescence analysis, the elemental composition (in moles) of the zeolite was determined to be Si/(Fe + Al) = 18.9, Al/(Fe + Al) = 0.54, Fe/(Fe + Al) = 0.46 (Table 3).

### Method for preparing Fe-Al- MFI/SiO₂ catalyst

The powdered Na-type Fe-Al-MFI zeolite synthesized according to the above-described process was used to prepare a proton-type Fe-Al-MFI zeolite/silica composite catalyst (i.e., Fe-Al-MFI/SiO₂ catalyst) in the same manner as Example 5. The composition (in weight) of this catalyst was determined to be 65/35 [wt%/wt%] Al-MFI/SiO₂ by X-ray fluorescence analysis (Table 3).

### <Method of performance evaluation test for Fe-Al-MFI/SiO₂ catalyst>

The performance evaluation test for the above-described Fe-Al-MFI/SiO₂ catalyst was conducted in the same manner as Example 7 (Table 5). The results are shown in Table 7 as described later.

### (Example 9)

### <Method for preparing Fe-Al-MFI/SiO₂ catalyst>

### Method for synthesizing Fe-Al-MFI zeolite

A powdered Na-type MFI zeolite containing Fe and Al (i.e., Fe-Al-MFI zeolite) was synthesized in the same manner as Example 3. Through X-ray fluorescence analysis, the elemental composition (in moles) of the zeolite was determined to be Si/(Fe + Al) = 19.4, Al/(Fe + Al) = 0.72, Fe/(Fe + Al) = 0.28 (Table 3).

### Method for preparing Fe-Al- MFI/SiO₂ catalyst

The powdered Na-type Fe-Al-MFI zeolite synthesized according to the above-described process was used to prepare a proton-type Fe-Al-MFI zeolite/silica composite catalyst (i.e., Fe-Al-MFI/SiO₂ catalyst) in the same manner as Example 3. The composition (in weight) of this catalyst was determined to be 65/35 [wt%/wt%] Fe-Al-MFI/SiO₂ by X-ray fluorescence analysis (Table 3).

### <Method of performance evaluation test for Fe-Al-MFI/SiO₂ catalyst>

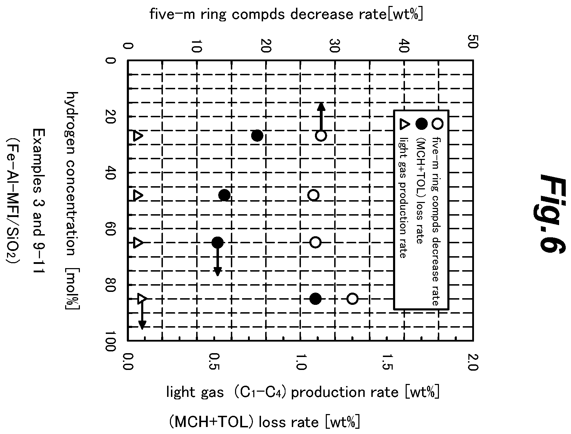

The performance evaluation test for the above-described Fe-Al-MFI/SiO₂ catalyst was conducted in the same manner as Example 3 except that hydrogen was supplied at a flow rate of 0.05 L/min (48 mol%) (Table 4). The results are shown in Figure 6 and Table 8 as described later.

### (Example 10)

### <Method for preparing Fe-Al-MFI/SiO₂ catalyst>

### Method for synthesizing Fe-Al-MFI zeolite

A powdered Na-type MFI zeolite containing Fe and Al (i.e., Fe-Al-MFI zeolite) was synthesized in the same manner as Example 3. Through X-ray fluorescence analysis, the elemental composition (in moles) of the zeolite was determined to be Si/(Fe + Al) = 19.4, Al/(Fe + Al) = 0.72, Fe/(Fe + Al) = 0.28 (Table 3).

### Method for preparing Fe-Al- MFI/SiO₂ catalyst

The powdered Na-type Fe-Al-MFI zeolite synthesized according to the above-described process was used to prepare a proton-type Fe-Al-MFI zeolite/silica composite catalyst (i.e., Fe-Al-MFI/SiO₂ catalyst) in the same manner as Example 3. The composition (in weight) of this catalyst was determined to be 65/35 [wt%/wt%] Al-MFI/SiO₂ by X-ray fluorescence analysis (Table 3).

### <Method of performance evaluation test for Fe-Al-MFI/SiO₂ catalyst>

The performance evaluation test for the above-described Fe-Al-MFI/SiO₂ catalyst was conducted in the same manner as Example 3 except that hydrogen was supplied at a flow rate of 0.10 L/min (65 mol%) (Table 4). The results are shown in Figure 6 and Table 8 as described later.

### (Example 11)

### <Method for preparing Fe-Al-MFI/SiO₂ catalyst>

### Method for synthesizing Fe-Al-MFI zeolite

A powdered Na-type MFI zeolite containing Fe and Al (i.e., Fe-Al-MFI zeolite) was synthesized in the same manner as Example 3. Through X-ray fluorescence analysis, the elemental composition (in moles) of the zeolite was determined to be Si/(Fe + Al) = 19.4, Al/(Fe + Al) = 0.72, Fe/(Fe + Al) = 0.28 (Table 3).

### Method for preparing Fe-Al- MFI/SiO₂ catalyst

The powdered Na-type Fe-Al-MFI zeolite synthesized according to the above-described process was used to prepare a proton-type Fe-Al-MFI zeolite/silica composite catalyst (i.e., Fe-Al-MFI/SiO₂ catalyst) in the same manner as Example 3. The composition (in weight) of this catalyst was determined to be 65/35 [wt%/wt%] Al-MFI/SiO₂ by X-ray fluorescence analysis (Table 3).

### <Method of performance evaluation test for Fe-Al-MFI/SiO₂ catalyst>

The performance evaluation test for the above-described Fe-Al-MFI/SiO₂ catalyst was conducted in the same manner as Example 3 except that hydrogen was supplied at a flow rate of 0.30 L/min (85 mol%) (Table 4). The results are shown in Figure 6 and Table 8 as described later.

### (Comparative Example 1)

### <Method for preparing Al-BETA/SiO₂ catalyst>

### Method for synthesizing Al-BETA zeolite

Commercially available Al-BETA zeolite (H-BEA-50 Powder, Süd-Chemi Catalyst Co., Ltd., Si/Al ratio in moles: approx. 20) was used as the zeolite sample (Table 3).

### Method for preparing Al-BETA/SiO₂ catalyst

Using the powdered Na-type Al-BETA zeolite synthesized according to the above-described process, a proton-type Al-BETA zeolite/silica composite catalyst (hereafter referred to as the "Al-BETA/SiO₂ catalyst") was prepared in the same way as Examples 1 and 7. The composition (in weight) of this catalyst was determined to be 65/35 [wt%/wt%] Al-BETA/SiO₂ by X-ray fluorescence analysis (Table 3).

### <Method of performance evaluation test for Al-BETA/SiO₂ catalyst>

The performance evaluation test for the above-described Al-BETA/SiO₂ catalyst was conducted in the same manner as Example 1 (Table 4). The results are shown in Figure 5 and Table 6 as described later.

### (Examples 1-6)

As shown in Figure 5(A) (also see Table 6), in Example 1 (Al-MFI/SiO₂ catalyst), when the temperature of the catalyst layer was 350°C, the five-membered ring compound decrease rate was about 40 wt%. The hydrogen carrier loss rate (MCH+TOL) was about 3.5 wt%. The hydrogen carrier loss rate was kept low compared to the five-membered ring compound decrease rate. The light gas production rate, i.e., the rate of production of light gas components (C₁-C₄) produced by the decomposition of the five-membered ring compound was about 0.3 wt%. In this way, the results showed that the hydrocracking reaction using the Al-MFI/SiO₂ catalyst can selectively decrease five-membered ring compounds while keeping the hydrogen carrier loss rate low. In Comparative Example 1 (Al-BETA/SiO₂ catalyst), the five-membered ring compound decrease rate at a catalyst bed temperature of 350°C was less than 0.1 wt%, and the results showed that the catalyst had substantially no decomposition activity.

In Example 2 (Ga-MFI/SiO₂ catalyst), the rate of decrease in five-membered ring compounds (28.7 wt%) was slightly lower than in Example 1, and the hydrogen carrier (MCH+TOL) loss rate (0.7 wt%) was further lowered. In this way, the results showed that the hydrocracking reaction using the Ga-MFI/SiO2 catalyst can selectively decrease five-membered ring compounds while keeping the hydrogen carrier loss rate in a more effective manner.

In Examples 3-6 (Fe-Al-MFI/SiO₂ catalysts), the higher the ratio of iron in the zeolite that forms the catalyst ("Fe/T" and "Al/T" in Table 3), the lower the five-membered ring compound decrease rates (27.9 wt%, 21.2 wt%, 20.0 wt%, and 11.3 wt%, respectively). However, in Examples 3-6, the hydrogen carrier (MCH+TOL) loss rates were kept lower than in Example 1. In this way, the results showed that the hydrocracking reaction using the Fe-Al-MFI/SiO₂ catalysts can selectively decrease five-membered ring compounds while keeping the hydrogen carrier loss rate low in a more effective manner. This can be understood that, with the increase in the rate of iron, the acid strengths of acid sites in the zeolite are weakened, resulting in the suppression of the hydrocracking reaction of five-membered ring compounds.

As shown in Figures 5(B) and (C) (also see Table 6), Examples 1-6 showed performance evaluation data at catalyst layer temperatures of 375°C and 400°C. As a result, when the same type of catalyst was used, with the increase in the catalyst layer temperature, the five-membered ring compound decrease rate also increased. In addition, with the increase in the catalyst layer temperature, the hydrogen carrier loss rate also increased, but was not more than about 8 wt% at the maximum for all the catalysts. In Comparative Example 1 (Al-BETA/SiO₂ catalyst), the five-membered ring compound decrease rate at a catalyst layer temperature of 375°C was less than 0.1 wt%, and was about 4.8 wt% at a catalyst layer temperature of 400°C. Therefore, the results showed that the catalyst of Comparative Example 1 has a five-membered ring compound decomposition activity that is much lower than those of Examples 1-6 (Al-MFI/SiO₂ catalyst, Ga-MFI/SiO₂ catalyst, Fe-Al-MFI/SiO₂ catalyst).

**[Table 6]**

| | reaction temperature [°C] | five-m ring compd decrease rate [wt%] | light gas (C₁-C₄) production rate [wt%] | MCH reaction rate [wt%] | TOL reaction rate [wt%] | (MCH+TOL) loss rate [wt%] |
|---|---|---|---|---|---|---|
| C. Ex. 1 | 350 | < 0.1 | 0.03 | 12.6 | 0.7 | 1.0 |
| | 375 | < 0.1 | 0.06 | 18.6 | 1.3 | 1.7 |
| | 400 | 4.8 | 0.10 | 21.5 | 1.6 | 2.0 |
| Ex. 1 | 350 | 40.9 | 0.27 | 28.6 | 2.9 | 3.5 |
| | 375 | 58.6 | 0.51 | 47.1 | 5.9 | 6.8 |
| | 400 | 67.6 | 0.59 | 52.8 | 6.5 | 7.6 |
| Ex. 2 | 350 | 28.7 | 0.08 | 5.5 | 0.6 | 0.7 |
| | 375 | 42.2 | 0.20 | 14.8 | 1.5 | 1.8 |
| | 400 | 60.5 | 0.45 | 32.1 | 2.7 | 3.4 |
| Ex. 3 | 350 | 27.9 | 0.07 | 10.4 | 0.5 | 0.8 |
| | 375 | 42.4 | 0.18 | 19.2 | 1.8 | 2.2 |
| | 400 | 59.3 | 0.37 | 35.1 | 3.5 | 4.2 |
| Ex. 4 | 350 | 21.2 | 0.04 | 5.2 | 0.3 | 0.4 |
| | 375 | 32.3 | 0.11 | 12.7 | 1.2 | 1.4 |
| | 400 | 52.7 | 0.26 | 27.1 | 2.3 | 2.9 |
| Ex. 5 | 350 | 20.0 | 0.04 | 4.7 | 0.3 | 0.4 |
| | 375 | 32.2 | 0.08 | 10.3 | 0.8 | 1.0 |
| | 400 | 49.4 | 0.22 | 21.7 | 1.8 | 2.2 |
| Ex. 6 | 350 | 11.3 | 0.01 | 1.9 | 0.2 | 0.2 |
| | 375 | 17.9 | 0.03 | 4.0 | 0.3 | 0.4 |
| | 400 | 28.1 | 0.08 | 7.7 | 0.7 | 0.8 |

### (Examples 7-8)

In Example 7 (Al-MFI/SiO₂ catalyst), the performance of the catalyst was evaluated under the same reaction conditions as in Examples 1-6, using a benzene solution as the reaction raw material. As shown in Table 7, when the catalyst bed temperature was 350°C, the five-membered ring compound (MCP) decrease rate was about 35.5wt%. The hydrogen carrier (CH+Bz) loss rate was about 2.6wt%, and the light gas production rate, i.e., the rate of production of light gas components (C₁-C₄) produced by the decomposition of the five-membered ring compounds was about 0.14wt%. At a catalyst bed temperature of 400°C, with the increase in the temperature, the rate of decrease of the five-membered ring compounds increased to about 73 wt%, and the hydrogen carrier loss rate also increased (Table 7), but was not more than 9 wt%. Therefore, the results showed that the hydrocracking reaction using the Al-MFI/SiO₂ catalyst can selectively decrease the five-membered ring compounds (MCPs) produced as byproducts of the cyclohexane dehydrogenation reaction while keeping the hydrogen carrier loss rate low.

In Example 8 (Fe-Al-MFI/SiO₂ catalyst), the five-membered ring compound decrease rates (14.7 wt% at 350°C, 38.0 wt% at 400°C) were lower than in Example 7, but the hydrogen carrier (CH+Bz) loss rates (0.3 wt% at 350°C, 1.8 wt% at 400°C) were lower. In this way, the results showed that the hydrocracking reaction using the Fe-Al-MFI/SiO₂ catalyst can selectively decrease the five-membered ring compounds (MCP) produced as byproducts of the cyclohexane dehydrogenation reaction, while keeping the hydrogen carrier loss rate in a more effective manner.

**[Table 7]**

| | reaction temperature [°C] | five-m ring compd decrease rate [wt%] | light gas (C₁-C₄) production rate [wt%] | CH reaction rate [wt%] | Bz reaction rate [wt%] | (CH + Bz) loss rate [wt%] |
|---|---|---|---|---|---|---|
| Ex. 7 | 350 | 35.5 | 0.14 | 21.5 | 2.2 | 2.6 |
| | 400 | 72.8 | 0.46 | 53.0 | 7.6 | 8.6 |
| Ex. 8 | 350 | 14.7 | 0.01 | 3.0 | 0.2 | 0.3 |
| | 400 | 38.0 | 0.13 | 16.1 | 1.5 | 1.8 |

As described above, the inventors discovered that the hydrocracking reaction using the zeolite composite catalyst (Al-MFI/SiO₂ catalyst, Ga-MFI/SiO2 catalyst, or Fe-Al-MFI/SiO₂ catalyst) according to the first embodiment can selectively decrease five-membered ring compounds while keeping the hydrogen carrier loss rates low in a more effective manner when the temperature of the catalyst layer is set to be in the range of 250°C to 400°C. It was also found that, when the hydrogen carrier is the cyclohexane dehydrogenation/benzene hydrogenation system, the effects of the zeolite composite catalyst of the present invention are effective as in the case of the methylcyclohexane dehydrogenation/toluene hydrogenation system. The temperature of the catalyst bed in the hydrocracking reaction may be lowered to around 200°C as long as the five-membered ring compound decrease rate is acceptable. However, when the temperature of the catalyst bed is lowered, the zeolite composite catalyst would not achieve sufficient catalytic performance (such as the five-membered ring compound decrease rate). For this reason, the temperature of the catalyst bed is preferably set to 250°C or greater. Moreover, the temperature of the catalyst bed in the hydrocracking reaction may be raised to around 450°C as long as the increase in the hydrogen carrier loss rate is acceptable. However, since the increase in the temperature of the catalyst bed can cause some problems such as coking may occur, the temperature of the catalyst bed is preferably set to 400°C or less.

### (Examples 9-11)

The test for determining the effect of a concentration of hydrogen in the reaction raw material supplied to the fixed-bed circulating reactor for the catalyst of Example 3 (Fe-Al-MFI/SiO₂ catalyst) using the following procedure. The hydrogen concentration in the reaction raw material was 27 mol% (hydrogen flow rate 0.02 L/min) in Example 3, 48 mol% (hydrogen flow rate 0.05 L/min) in Example 9, 65 mol% (hydrogen flow rate 0.10 L/min) in Example 10, and 85 mol% (hydrogen flow rate 0.30 L/min) in Example 11. The indicators of catalyst performance were determined in the same manner as in Example 3. (The results are shown in Figure 6 and Table 8.)

As shown in Figure 6 (also see Table 8), in the range of hydrogen concentrations of 27-65 mol% (Examples 3, 9, and 10), the variation in the hydrogen concentration had substantially no effect on the five-membered ring compound decrease rate. In the range of hydrogen concentrations of 27-65 mol%, the results showed that the hydrogen carrier loss rate was on a slight decreasing trend with increasing hydrogen concentration, but was not significantly affected by the variation. When a hydrogen concentration was 85 mol% (Example 11), the five-membered ring compound decrease rate and the hydrogen carrier loss rate both increased compared to Examples 3, 9 and 10, but were still within an acceptable range. In this way, the results showed that the level of the hydrogen concentration in the reaction raw material supplied to the fixed-bed circulating reactor has no significant effect on the five-membered ring compound decrease rate and the hydrogen carrier loss rate (catalytic performance) due to hydrocracking reactions.

**[Table 8]**

| | flow rate (concentration) of hydrogen [L/min (mol%)] | five-m ring compd decrease rate [wt%] | light gas (C₁-C₄) production rate [wt%] | MCH reaction rate [wt%] | TOL reaction rate [wt%] | (MCH+TOL) loss rate [wt%] |
|---|---|---|---|---|---|---|
| Ex. 3 | 0.02 (27) | 27.9 | 0.07 | 10.4 | 0.52 | 0.75 |
| Ex. 9 | 0.05 (48) | 26.7 | 0.05 | 6.8 | 0.41 | 0.56 |
| Ex. 10 | 0.10 (65) | 27.1 | 0.03 | 6.0 | 0.39 | 0.52 |
| Ex. 11 | 0.30 (85) | 32.4 | 0.02 | 4.5 | 1.01 | 1.09 |

### <Second Embodiment>

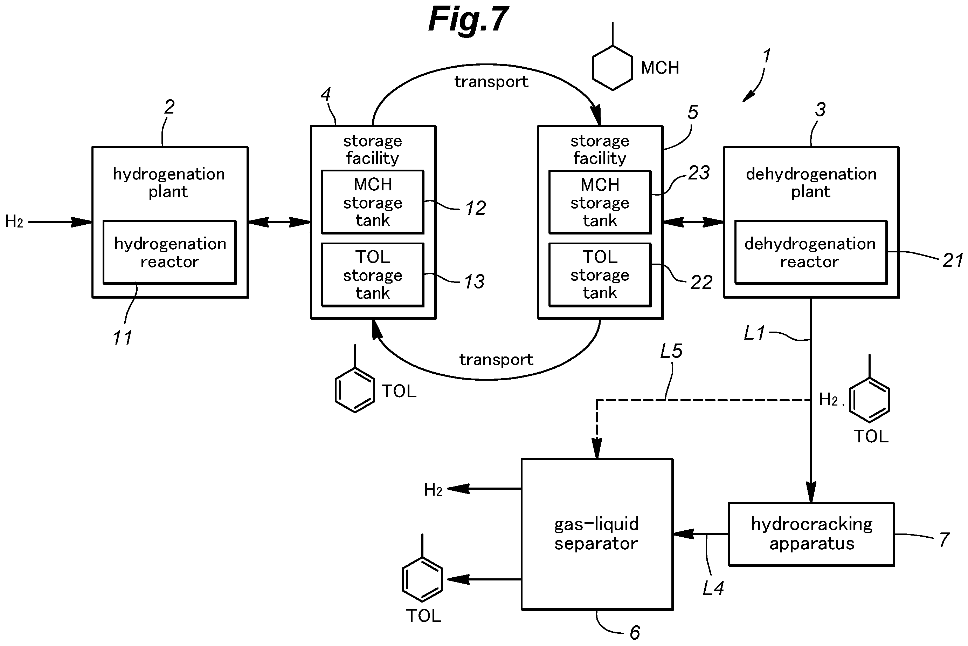

Figure 7 shows a schematic configuration of a hydrogen storage and transportation system according to a second embodiment of the present invention. In Figure 7, the same reference numerals denote similar features or components as in the first embodiment, and the detailed description of those features or components will not be repeated for the second embodiment.

The hydrogen storage and transportation system 1 of the second embodiment includes a gas-liquid separator 6 and a hydrocracking apparatus 7, as in the above-described first embodiment. However, in the second embodiment, the hydrocracking apparatus 7 is connected to the discharge line L1 for discharging reaction products of the dehydrogenation reactor 21. In other words, in the first embodiment, there is no gas-liquid separator 6 between the dehydrogenation reactor 21 and the hydrocracking apparatus 7 (i.e., the reaction products are not separated into gas and liquid). In the second embodiment, all or some of the reaction products of the dehydrogenation reactor 21 are supplied to the hydrocracking apparatus 7 in the form of gas from a reaction vessel. The hydrocracking apparatus 7 uses a hydrogenolysis catalyst to selectively hydrocrack the five-membered ring compounds that have been mixed in as impurities in the reaction products of the dehydrogenation reactor 21, to convert the five-membered ring compounds into linear light hydrocarbons. Since, in the dehydrogenation reaction of methylcyclohexane, 1 mol of toluene and 3 mol of hydrogen are produced from 1 mol of methylcyclohexane, a ratio of the amounts of gaseous hydrogen and liquid toluene (in moles) in the reaction products of the dehydrogenation reactor 21 is approximately 3:1. The products of the dehydrogenation reaction, from which the five-membered ring compounds have been removed (decreased), are supplied to the gas-liquid separator 6 via a transportation line L4.

The gas-liquid separator 6 separates the products of the dehydrogenation reaction, from which the five-membered ring compounds have been removed, into a gas phase consisting mainly of hydrogen and a liquid phase consisting mainly of toluene. The liquid containing toluene in the liquid phase is supplied to the TOL storage tank 22 and stored therein. In addition to hydrogen, the separated gas phase contains at least some of the light hydrocarbons in the form of chains (gases) produced by the hydrocracking of the five-membered ring compounds. Such light hydrocarbons can be separated from hydrogen using known technology.

In the hydrogen storage and transportation system 1, parts of the reaction products (including hydrogen, toluene, and five-membered ring compounds) from the dehydrogenation reactor 21 can be excluded from the subject of hydrocracking i.e., not being supplied to the hydrocracking apparatus 7. For example, in the hydrogen storage and transportation system 1, a branch line L5 is provided to branch from the discharge line L1, and at least a part (0-100%) of the reaction products of the dehydrogenation reactor 21 may be supplied to the gas-liquid separator 6 via the branch line L5. As in the first embodiment, the amount (percentage) of the liquid excluded from the subject of hydrocracking can be determined based on the concentration of the five-membered ring compounds mixed in toluene, the performance of the hydrocracking apparatus 7, and the amount of hydrogen carrier required by the system.

The hydrogenolysis catalysts of the second embodiment and methods for hydrocracking a five-membered ring compound using the catalyst of preferred embodiments will be described with reference to Examples 12-24. It is assumed that a reaction raw material, which is a product of the dehydrogenation reaction, was the reaction product of the dehydrogenation reactor 21, and the reaction raw material was a mixture of a toluene solution shown in Table 1 and hydrogen (75mol%).

Table 9 shows a list of the various catalyst samples used in Examples 12-24. Table 10 shows a list of conditions of a performance evaluation test for each of the catalyst samples in Examples 12-15. As will be described in detail later, in Example 12, an Al-MFI/SiO₂ catalyst was used in the same manner as in Examples 1 and 7. In Examples 13, 16-21, Ga-MFI/SiO₂ catalysts were used in the same manner as in Example 2. In Example 14, an Fe-Al-MFI/SiO₂ catalyst was used in the same manner as in Examples 3 and 9-11. Examples 15 and 22-24 used Ga-MFI/SiO₂ catalysts (Si/T = 10.1) with higher acidities than the Ga-MFI/SiO₂ catalyst (Si/T = 17.3) used in Example 2. The items in Table 9 are the same as those in Table 3.

**[Table 9]**

| | catalyst | catalyst composition [mol/mol] | | | | binder | zeolite/binder MR [wt%/wt%] |
|---|---|---|---|---|---|---|---|
| | | Si/T | Al/T | Ga/T | Fe/T | | |
| Ex. 12 | Al-MFI/SiO₂ | 21.3 | 1.00 | 0.00 | 0.00 | SiO₂ | 65/35 |
| Ex. 13,16-21 | Ga-MFI/SiO2 | 17.3 | 0.06 | 0.94 | 0.00 | SiO₂ | 65/35 |
| Ex. 14 | Fe-Al-MFI/SiO₂ | 19.4 | 0.72 | 0.00 | 0.28 | SiO₂ | 65/35 |
| Ex. 15,22-24 | Ga-MFI/SiO₂ | 10.1 | 0.02 | 0.98 | 0.00 | SiO₂ | 65/35 |

**[Table 10]**

| | reaction temperature [°C] | reaction pressure [MPa] | LHSV of raw material solution [h⁻¹] | flow rate (concentration) of hydrogen [L/min (mol%)] |
|---|---|---|---|---|
| Ex. 12 | 300 | 0.30 | 6.0 | 0.16 (75) |
| | 325 | 0.30 | 6.0 | 0.16 (75) |
| | 350 | 0.30 | 6.0 | 0.16 (75) |
| | 375 | 0.30 | 6.0 | 0.16 (75) |
| | 400 | 0.30 | 6.0 | 0.16 (75) |
| Ex. 13 | 300 | 0.30 | 6.0 | 0.16 (75) |
| | 325 | 0.30 | 6.0 | 0.16 (75) |
| | 350 | 0.30 | 6.0 | 0.16 (75) |
| | 375 | 0.30 | 6.0 | 0.16 (75) |
| | 400 | 0.30 | 6.0 | 0.16 (75) |
| Ex. 14 | 300 | 0.30 | 6.0 | 0.16 (75) |
| | 325 | 0.30 | 6.0 | 0.16 (75) |
| | 350 | 0.30 | 6.0 | 0.16 (75) |
| | 375 | 0.30 | 6.0 | 0.16 (75) |
| | 400 | 0.30 | 6.0 | 0.16 (75) |
| Ex. 15 | 325 | 0.30 | 6.0 | 0.16 (75) |
| | 350 | 0.30 | 6.0 | 0.16 (75) |
| | 375 | 0.30 | 6.0 | 0.16 (75) |

### (Example 12)

### <Method of performance evaluation test for Al-MFI/SiO₂ catalyst>

The performance evaluation test for the above-described Al-MFI/SiO₂ catalyst was conducted using the following procedure. The Al-MFI/SiO₂ catalyst was packed into a fixed-bed, circulating-type reactor as a 2.32 mL catalyst bed. The reaction raw material (Table 1) was supplied into the reactor at a flow rate of 0.235 mL/min (LHSV 6 h⁻¹) together with hydrogen at a flow rate of 0.16 L/min (75 mol%), and the hydrocracking reaction of the reaction raw material was caused at a pressure of 0.30 MPa and average temperatures of the catalyst bed (i.e., reaction temperatures) of 300°C, 325°C, 350°C, 375°C, and 400°C for a predetermined time (Table 10). Two hours after the hydrocracking reaction started at each catalyst bed temperature, the gas-phase and liquid-phase products were sampled. Then, the five-membered ring compound decrease rate (wt%), the MCH+TOL (hydrogen carrier) loss rate, and the light gas production rate (wt%), i.e., the rate of production of light gas components (C₁-C₄) were determined based on the results of FID-GC (flame ionization detector gas chromatograph) analysis, and these were used as indicators of catalyst performance. The results are shown in Table 11 as described later.

### (Example 13)

### <Method of performance evaluation test for Ga-MFI/SiO₂ catalyst>

The performance evaluation test for the above-described Ga-MFI/SiO₂ catalyst was conducted in the same manner as Example 12 (Table 10). The results are shown in Table 11 as described later.

### (Example 14)

### <Method of performance evaluation test for Fe-Al-MFI/SiO₂ catalyst>

The performance evaluation test for the above-described Fe-Al-MFI/SiO₂ catalyst was conducted in the same manner as Example 12 (Table 10). The results are shown in Table 11 as described later.

### (Example 15)

### <Method for preparing Ga-MFI/SiO₂ catalyst>

### Method for synthesizing Ga-MFI zeolite

A powdered Na-type MFI zeolite containing Ga (hereinafter referred to as Ga-MFI zeolite) was synthesized in the same manner as Example 1 except that a solution comprising 58.9g colloidal silica (30.6 wt% SiO₂, 0.4 wt% Na₂O, 69.0 wt% H₂O), and 2.99g sodium hydroxide was prepared as Solution A, and a solution comprising 7.62g gallium nitrate pentahydrate, 15.49g tetrapropylammonium bromide, and 185.18g purified water was prepared as Solution B. Through X-ray fluorescence analysis, the elemental composition (in moles) of the zeolite was determined to be Si/Ga = 10.1 (Table 9).

### Method for preparing Ga-MFI/SiO₂ catalyst

The powdered Na-type Ga-MFI zeolite synthesized according to the above-described process was used to prepare a proton-type Ga-MFI zeolite/silica composite catalyst (hereafter referred to as "Ga-MFI/SiO₂ catalyst") in the same manner as Example 1. The composition (in weight) of this catalyst was determined to be 65/35 [wt%/wt%] Ga-MFI/SiO₂ by X-ray fluorescence analysis (Table 9).

### <Method of performance evaluation test for Ga-MFI/SiO₂ catalyst>

The performance evaluation test for the above-described Ga-MFI/SiO₂ catalyst was conducted in the same manner as Example 12 (Table 10) except that the average temperatures of the catalyst bed are set to 350°C, 375°C, and 400°C. The results are shown in Table 11 as described later.

### (Example 16)

### <Method of performance evaluation test for Ga-MFI/SiO₂ catalyst>

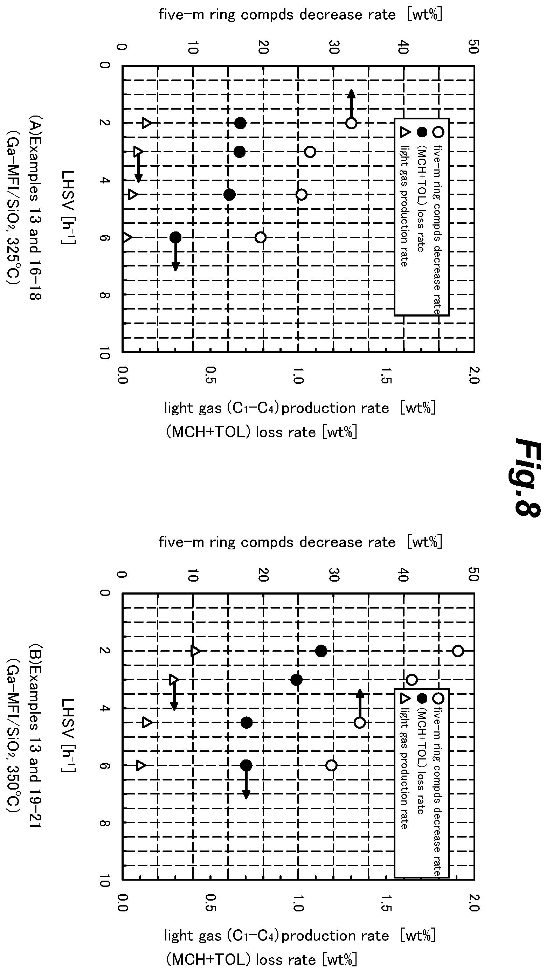

The performance evaluation test for the above-described Ga-MFI/SiO₂ catalyst (the catalyst sample used in Example 13) was conducted using the following procedure. The Ga-MFI/SiO₂ catalyst was packed into a fixed-bed, circulating-type reactor as a 4.65 mL catalyst bed. The reaction raw material (Table 1) was supplied into the reactor at a flow rate of 0.35 mL/min (LHSV 4.5 h⁻¹) together with hydrogen at a flow rate of 0.24 L/min (75 mol%), and the hydrocracking reaction of the reaction raw material was caused at a pressure of 0.30 MPa and an average temperature of the catalyst bed of 325°C for a predetermined time (Table 12). Two hours after the hydrocracking reaction started, the gas-phase and liquid-phase products were sampled. Then, the five-membered ring compound decrease rate (wt%), the MCH+TOL (hydrogen carrier) loss rate, and the light gas production rate (wt%), i.e., the rate of production of light gas components (C₁-C₄) were determined based on the results of FID-GC (flame ionization detector gas chromatograph) analysis, and these were used as indicators of catalyst performance. The results are shown in Figure 8 and Table 12 as described later.

### (Example 17)

### <Method of performance evaluation test for Ga-MFI/SiO₂ catalyst>

The performance evaluation test for the above-described Ga-MFI/SiO₂ catalyst (the catalyst sample used in Example 13) was conducted in the same manner as Example 16 (Table 12) except that the reaction raw material (Table 1) was supplied into the reactor at a flow rate of 0.235 mL/min (LHSV 3.0 h⁻¹) together with hydrogen at a flow rate of 0.16 L/min (75 mol%). The results are shown in Figure 8 and Table 12 as described later.

### (Example 18)

### <Method of performance evaluation test for Ga-MFI/SiO₂ catalyst>

The performance evaluation test for the above-described Ga-MFI/SiO₂ catalyst (the catalyst sample used in Example 13) was conducted in the same manner as Example 16 (Table 12) except that the reaction raw material (Table 1) was supplied into the reactor at a flow rate of 0.155 mL/min (LHSV 2.0 h⁻¹) together with hydrogen at a flow rate of 0.11 L/min (75 mol%). The results are shown in Figure 8 and Table 12 as described later.

### (Example 19)

### <Method of performance evaluation test for Ga-MFI/SiO₂ catalyst>

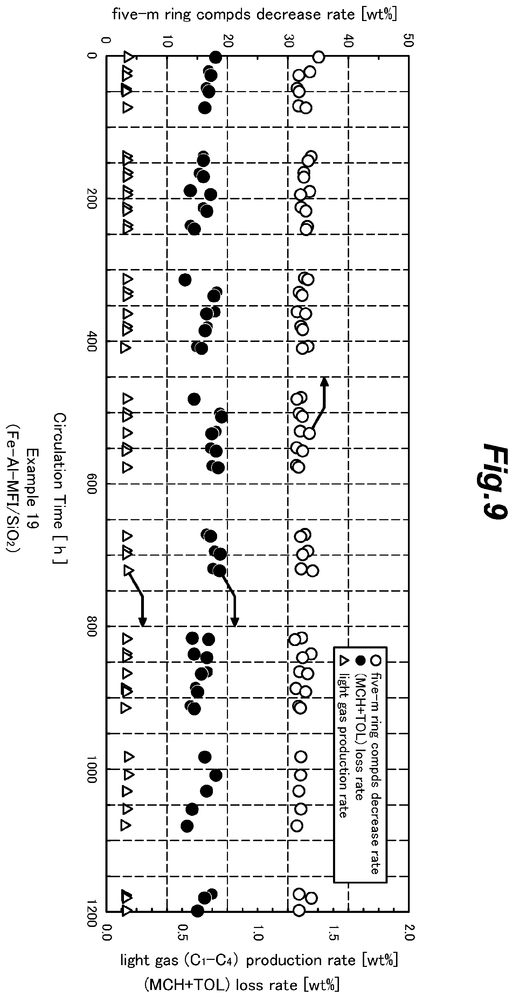

The performance evaluation test for the above-described Ga-MFI/SiO₂ catalyst (the catalyst sample used in Example 13) was conducted in the same manner as Example 16 (Table 12) except that an average temperature of the catalyst bed is set to 350°C. The results are shown in Figure 8 and Table 12 as described later. In addition, in order to evaluate the stability of the catalyst sample, the Ga-MFI/SiO2 catalyst was packed into a fixed-bed, circulating-type reactor as a 2.32 mL catalyst bed. The reaction raw material (Table 1) was supplied into the reactor at a flow rate of 0.235 mL/min (LHSV 4.5 h⁻¹) together with hydrogen at a flow rate of 0.12 L/min (75 mol%), and the hydrocracking reaction of the reaction raw material was caused at a pressure of 0.30 MPa and an average temperature of the catalyst bed of 350°C for 1,200 hours. The results are shown in Figure 9 as described later.

### (Example 20)

### <Method of performance evaluation test for Ga-MFI/SiO₂ catalyst>

The performance evaluation test for the above-described Ga-MFI/SiO₂ catalyst (the catalyst sample used in Example 13) was conducted in the same manner as Example 17 (Table 12) except that an average temperature of the catalyst bed was set to 350°C. The results are shown in Figure 8 and Table 12 as described later.

### (Example 21)

### <Method of performance evaluation test for Ga-MFI/SiO₂ catalyst>

The performance evaluation test for the above-described Ga-MFI/SiO₂ catalyst (the catalyst sample used in Example 13) was conducted in the same manner as Example 18 (Table 12) except that an average temperature of the catalyst bed was set to 350°C. The results are shown in Figure 8 and Table 12 as described later.

### (Example 22)

### <Method of performance evaluation test for Ga-MFI/SiO₂ catalyst>

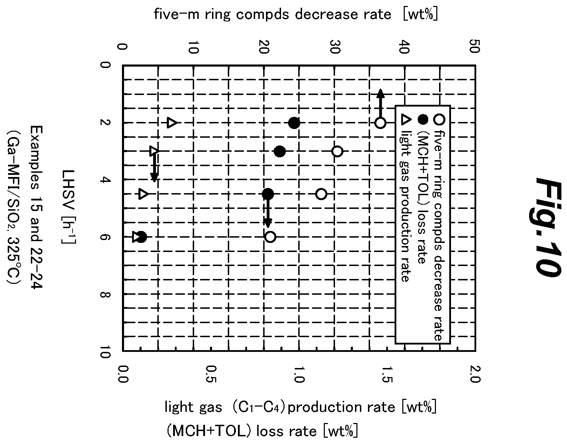

The performance evaluation test for the above-described Ga-MFI/SiO₂ catalyst (the catalyst sample used in Example 15) was conducted in the same manner as Example 16 (Table 13) except that an average temperature of the catalyst bed is set to 350°C. The results are shown in Figure 10 and Table 13 as described later.

### (Example 23)

### <Method of performance evaluation test for Ga-MFI/SiO₂ catalyst>

The performance evaluation test for the above-described Ga-MFI/SiO₂ catalyst (the catalyst sample used in Example 15) was conducted in the same manner as Example 17 (Table 13) except that an average temperature of the catalyst bed is set to 350°C. The results are shown in Figure 10 and Table 13 as described later.

### (Example 24)

### <Method of performance evaluation test for Ga-MFI/SiO₂ catalyst>

The performance evaluation test for the above-described Ga-MFI/SiO₂ catalyst (the catalyst sample used in Example 15) was conducted in the same manner as Example 18 (Table 13) except that an average temperature of the catalyst bed is set to 350°C. The results are shown in Figure 10 and Table 13 as described later.

As shown in Table 11, in Example 12 (Al-MFI/SiO₂ catalyst), when the temperature of the catalyst layer was set to be in the range of 300°C to 400°C, the five-membered ring compound decrease rate and the hydrogen carrier loss rate increased with the increase in the temperature of the catalyst layer. However, at all the temperatures, the hydrogen carrier loss rate was kept low compared to the five-membered ring compound decrease rate. In this way, the results of the second embodiment showed that the hydrocracking reactions using Al-MFI/SiO₂ catalysts can selectively decrease five-membered ring compounds while keeping the hydrogen carrier loss rate low.

In Example 13 (Ga-MFI/SiO₂ catalyst), when the temperature of the catalyst layer was set to be in the range of 300°C to 400°C, the five-membered ring compound decrease rate and the hydrogen carrier loss rate increased with the increase in the temperature of the catalyst layer, in a similar manner to Example 12. However, at all the temperatures, the hydrogen carrier loss rate was kept low compared to the five-membered ring compound decrease rate. Furthermore, in Example 13, the five-membered ring compound decrease rate at each temperature was lower than in Example 12, and the hydrogen carrier loss rate was lower than in Example 12. In this way, the results of the second embodiment showed that the hydrocracking reactions using Ga-MFI/SiO₂ catalysts can selectively decrease five-membered ring compounds while keeping the hydrogen carrier loss rate low.

In Example 14 (Fe-Al-MFI/SiO₂ catalyst), when the temperature of the catalyst layer was set to be in the range of 300°C to 400°C, the five-membered ring compound decrease rate and the hydrogen carrier loss rate increased with the increase in the temperature of the catalyst layer, in a similar manner to Examples 12 and 13. In Example 14, the five-membered ring compound decrease rate at each temperature was further lower than in Example 13 (except at 300°C), but the hydrogen carrier loss rate was kept low as in Example 13. However, at all the temperatures, the hydrogen carrier loss rate was kept low compared to the five-membered ring compound decrease rate. In this way, the results of the second embodiment showed that the hydrocracking reactions using Fe-Al-MFI/SiO₂ catalysts can selectively decrease five-membered ring compounds while keeping the hydrogen carrier loss rate low.

Example 15 used Ga-MFI/SiO₂ catalysts (Si/T = 10.1) with higher acidities than the Ga-MFI/SiO₂ catalyst (Si/T = 17.3) used in Example 13 (Tabel 9). When the temperature of the catalyst layer was set to be in the range of 325°C to 375°C, the five-membered ring compound decrease rate and the hydrogen carrier loss rate increased with the increase in the temperature of the catalyst layer, in a similar manner to Examples 12 to 14. However, the hydrogen carrier loss rate was equal to or greater than Example 12 and the five-membered ring compound decrease rate was kept lower than Examples 13 and 14. In this way, the results of the second embodiment showed that the hydrocracking reactions using Fe-Al-MFI/SiO₂ catalysts with higher acidities can decrease five-membered ring compounds in a more selective manner while keeping the hydrogen carrier loss rate low.

As described above, the inventors discovered that the hydrocracking reaction using the zeolite composite catalyst (Al-MFI/SiO₂ catalyst, Ga-MFI/SiO₂ catalyst, or Fe-Al-MFI/SiO₂ catalyst) according to the second embodiment can selectively decrease five-membered ring compounds while keeping the hydrogen carrier loss rates low in a more effective manner when the temperature of the catalyst layer is set to be in the range of 300°C to 400°C. In particular, the Ga-MFI/SiO₂ catalyst achieved a high five-membered ring compounds decrease rate equivalent to that achievable by the Al-MFI/SiO₂ catalyst, while also achieving a low hydrogen carrier loss rate equivalent to that achievable by the Fe-Al-MFI/SiO₂ catalyst. The temperature of the catalyst bed in the hydrocracking reaction may be lowered to around 200°C and increased to around 450°C as in the first embodiment.

**[Table 11]**

| | reaction temperature [°C] | five-m ring compd decrease rate [wt%] | light gas (C₁-C₄) production rate [wt%] | MCH reaction rate [wt%] | TOL reaction rate [wt%] | (MCH+TOL) loss rate [wt%] |
|---|---|---|---|---|---|---|
| Ex. 12 | 300 | 15.6 | 0.00 | 4.8 | 0.3 | 0.4 |
| | 325 | 21.0 | 0.03 | 7.8 | 0.5 | 0.7 |
| | 350 | 32.3 | 0.15 | 18.6 | 1.1 | 1.5 |
| | 375 | 46.9 | 0.38 | 19.3 | 2.4 | 2.8 |
| | 400 | 61.5 | 0.61 | 34.3 | 4.0 | 4.7 |
| Ex. 13 | 300 | 12.8 | 0.00 | 2.5 | 0.3 | 0.4 |
| | 325 | 19.6 | 0.04 | 5.5 | 0.2 | 0.3 |
| | 350 | 29.7 | 0.11 | 7.0 | 0.6 | 0.7 |
| | 375 | 43.5 | 0.41 | 19.0 | 0.9 | 1.3 |
| | 400 | 57.7 | 0.71 | 20.6 | 1.1 | 1.5 |
| Ex. 14 | 300 | 13.2 | 0.00 | 2.3 | 0.9 | 0.9 |
| | 325 | 18.6 | 0.00 | 5.5 | 0.7 | 0.8 |
| | 350 | 26.3 | 0.02 | 8.8 | 0.4 | 0.7 |
| | 375 | 33.2 | 0.11 | 14.1 | 0.7 | 1.0 |
| | 400 | 45.7 | 0.25 | 22.7 | 1.1 | 1.6 |
| Ex. 15 | 325 | 20.8 | 0.10 | 11.6 | < 0.1 | 0.1 |
| | 350 | 33.9 | 0.26 | 12.3 | < 0.1 | 0.2 |
| | 375 | 48.6 | 0.58 | 23.4 | 0.2 | 0.7 |

In Examples 16-18, the test for determining the effect of a time of contact between catalysts (Ga-MFI/SiO₂ catalysts) in Example 13 and the reaction raw material supplied to the fixed-bed circulating reactor at a catalyst bed temperature of 325°C. As shown in Figure 8(A) (also see Table 12), in Examples 16-18, the five-membered ring compound decrease rate and the hydrogen carrier loss rate increased with the decrease in LHSV (i.e. the increase in the contact time between the reaction raw material and the catalyst). However, the variation in the hydrogen carrier loss rate was still within an acceptable range. In this way, it was found that, even when the LHSV changed, the hydrocracking reaction using the Ga-MFI/SiO₂ catalysts according to the second embodiment can selectively decrease five-membered ring compounds at the temperature of the catalyst layer of 325°C.

In Examples 19-21, the test for determining the effect of a time of contact between catalysts and the reaction raw material supplied to the fixed-bed circulating reactor at a catalyst bed temperature of 350°C. As shown in Figure 8(B) (also see Table 12), in Examples 16-18, the five-membered ring compound decrease rate and the hydrogen carrier loss rate increased with the decrease in LHSV (i.e. the increase in the contact time between the reaction raw material and the catalyst) compared to at the catalyst bed temperature of 325°C, but the variation in the hydrogen carrier loss rate was still within an acceptable range. Furthermore, the long-term performance evaluation test under the conditions of LHSV of 4.5 h⁻¹ showed that the catalytic performance (the five-membered ring compound decrease rate, hydrogen carrier loss rate, and light gas production rate) remained stable for about 1,200 hours (Figure 9). In this way, it was found that, even when the LHSV changed, the hydrocracking reaction using the Ga-MFI/SiO₂ catalysts according to the second embodiment can selectively decrease five-membered ring compounds at the temperature of the catalyst layer of 350°C. The results in Figure 9 also showed that the catalytic performance remained stable for about 1,200 hours (Figure 9).

**[Table 12]**

| | LHSV of raw material solution [h⁻¹] | reaction temperature [°C] | five-m ring compd decrease rate [wt%] | light gas (C₁-C₄) production rate [wt%] | MCH reaction rate [wt%] | TOL reaction rate [wt%] | (MCH+TOL) loss rate [wt%] |
|---|---|---|---|---|---|---|---|
| Ex. 13 | 6.0 | 325 | 19.6 | 0.04 | 5.5 | 0.2 | 0.3 |
| Ex. 16 | 4.5 | 325 | 25.4 | 0.07 | 7.1 | 0.7 | 0.6 |
| Ex. 17 | 3.0 | 325 | 26.6 | 0.10 | 7.5 | 0.5 | 0.7 |
| Ex. 18 | 2.0 | 325 | 32.6 | 0.15 | 10.1 | 0.5 | 0.7 |
| Ex. 13 | 6.0 | 350 | 29.7 | 0.11 | 7.0 | 0.6 | 0.7 |
| Ex. 19 | 4.5 | 350 | 33.8 | 0.15 | 9.4 | 0.5 | 0.7 |
| Ex. 20 | 3.0 | 350 | 41.3 | 0.30 | 14.1 | 0.7 | 1.0 |
| Ex. 21 | 2.0 | 350 | 47.7 | 0.42 | 17.8 | 0.7 | 1.1 |

In Examples 22-24, the test for determining the effect of a time of contact between catalysts (Ga-MFI/SiO₂ catalysts) in Example 15 and the reaction raw material supplied to the fixed-bed circulating reactor at a catalyst bed temperature of 325°C. As shown in Figure 10 (also see Table 13), in Examples 22-24, the five-membered ring compound decrease rate and the hydrogen carrier loss rate increased with the decrease in LHSV (i.e. the increase in the contact time between the reaction raw material and the catalyst). However, the variation in the hydrogen carrier loss rate was still within an acceptable range. In this way, it was found that, even when the LHSV changed, the hydrocracking reaction using the Ga-MFI/SiO₂ catalysts (Si/T = 10.1) with higher acidities according to the second embodiment can selectively decrease five-membered ring compounds at the temperature of the catalyst layer of 325°C.

**[Table 13]**

| | LHSV of raw material solution [h⁻¹ ] | reaction temperature [°C] | five-m ring compd decrease rate [wt%] | light gas (C₁-C₄) production rate [wt%] | MCH reaction rate [wt%] | TOL reaction rate [wt%] | (MCH+TOL) loss rate [wt%] |
|---|---|---|---|---|---|---|---|
| Ex. 15 | 6.0 | 325 | 20.8 | 0.10 | 11.6 | < 0.1 | 0.1 |
| Ex. 22 | 4.5 | 325 | 28.0 | 0.13 | 8.7 | 0.7 | 0.8 |
| Ex. 23 | 3.0 | 325 | 30.3 | 0.18 | 10.4 | 0.6 | 0.9 |
| Ex. 24 | 2.0 | 325 | 36.3 | 0.29 | 12.4 | 0.7 | 1.0 |

Specific embodiments of the present invention are described above. However, those embodiments are non-limiting, and various modifications may be made to the embodiments without departing from the scope of the present invention. In zeolite composite catalysts and a method for hydrocracking a cyclic compound having a five-membered ring structure using the same according to the above-described embodiments, not all elements included therein are essential. Thus, at least the skilled person can make various modifications including elimination of some elements to the embodiments as appropriate.

For example, zeolite composite catalysts and methods for hydrocracking a cyclic compound having a five-membered ring structure using the catalysts, according to the present invention are not limited to the above-described configurations embodied in the hydrocracking apparatus 7 in the hydrogen storage and transportation system 1, and the zeolite composite catalysts and methods can be embodied in various forms of applications for hydrocracking a cyclic compound having a five-membered ring structure.

A zeolite with an MFI structure used as a zeolite composite catalyst of the present invention may also be configured to comprise aluminum and gallium. Such a zeolite with an MFI structure may be configured to comprise iron without aluminum. The so-configured zeolites used as catalysts for hydrocracking cyclic compounds with a five-membered ring structure also have an effect of achieving the same catalytic performance as the catalysts disclosed in the above-described embodiments.

### GLOSSARY

- 1: hydrogen storage and transportation system
- 2: hydrogenation plant
- 3: dehydrogenation plant
- 4: storage facility
- 5: storage facility
- 6: gas-liquid separator
- 7: hydrocracking apparatus
- 11: hydrogenation reactor
- 12: MCH storage tank
- 13: TOL storage tank
- 21: dehydrogenation reactor
- 22: TOL storage tank
- 23: MCH storage tank

## Claims

1. A zeolite composite catalyst used for hydrocracking a cyclic compound having a five-membered ring structure, the five-membered ring structure being included in an aromatic compound used as a hydrogen carrier, wherein the zeolite composite catalyst comprises at least one of aluminum, gallium, and iron, and wherein the zeolite composite catalyst comprises a zeolite with an MFI structure, and silica.

2. The zeolite composite catalyst as claimed in claim 1, wherein the zeolite with the MFI structure comprises gallium, and has an acid density of 5 to 200 where the acid density is determined as a ratio of an amount of silicon to an amount of gallium, the amounts being expressed in moles.

3. The zeolite composite catalyst as claimed in claim 1, wherein the zeolite with the MFI structure comprises iron and aluminum.

4. The zeolite composite catalyst as claimed in claim 3, wherein the zeolite has an acid density of 5 to 200 where the acid density is determined as a ratio of an amount of silicon to a sum of amounts of iron and aluminum, and also has an iron ratio of 0.1 to 0.9 where the iron ratio is a ratio of an amount of iron to a sum of amounts of iron and aluminum, the amounts being expressed in moles.

5. The zeolite composite catalyst as claimed in claim 1, wherein the zeolite with the MFI structure comprises aluminum, and has an acid density of 5 to 200 where the acid density is determined as a ratio of an amount of silicon to an amount of aluminum, the amounts being expressed in moles.

6. A method for hydrocracking a cyclic compound having a five-membered ring structure, the five-membered ring structure being included in an aromatic compound used as a hydrogen carrier, using the zeolite composite catalyst as claimed in any one of claims 1 to 5, the method comprising:
causing dehydrogenation of a saturated cyclic compound to produce, as reaction products, the aromatic compound and a cyclic compound having a five-membered ring structure as an impurity of the aromatic compound; and
hydrocracking at least part of the reaction products, using the zeolite composite catalyst.

7. The method as claimed in claim 6, wherein, during a reaction of the hydrocracking, a temperature of a catalyst bed is adjusted to 200°C to 450°C.

8. The method as claimed in claim 7, further comprising separating gas and liquid phases of the reaction products to produce a liquid phase product, and
wherein the step of hydrocracking using the zeolite composite catalyst comprises hydrocracking a mixture of the liquid phase product and hydrogen.

9. The method as claimed in claim 8, wherein the mixture contains hydrogen in an amount ranging from 5 mol% to 90 mol%.
